(19) Europäisches Patentamt
European Patent Office
Office européen des brevets

(11) EP 1 212 086 B1

(12) **EUROPEAN PATENT SPECIFICATION**

(45) Date of publication and mention of the grant of the patent:
**14.11.2007 Bulletin 2007/46**

(21) Application number: **99938639.4**

(22) Date of filing: **18.08.1999**

(51) Int Cl.:
***A61K 39/39*** *(2006.01)* ***A61K 38/02*** *(2006.01)*
***C07K 14/435*** *(2006.01)* ***C07K 14/78*** *(2006.01)*
***C07K 17/08*** *(2006.01)*

(86) International application number:
**PCT/KR1999/000460**

(87) International publication number:
**WO 2001/012222 (22.02.2001 Gazette 2001/08)**

(54) **IMMUNOLOGICAL TOLERANCE-INDUCTION AGENT**

AGENT ZUR INDUKTION EINER IMMUNOLOGISCHEN TOLERANZ

AGENT D'INDUCTION D'UNE TOLERANCE IMMUNOLOGIQUE

(84) Designated Contracting States:
**AT BE CH CY DE DK ES FI FR GB GR IE IT LI LU MC NL PT SE**

(43) Date of publication of application:
**12.06.2002 Bulletin 2002/24**

(73) Proprietor: **Industy-Academic Cooperation Foundation,**
**The Catholic University of Korea**
**Seocho-gu**
**Seoul**
**137-701 (KR)**

(72) Inventors:
- **KIM, Ho-Youn**
  **Seoul 138-737 (KR)**
- **PARK, Jong-Sang**
  **Shillim 9-Dong, Kwanak-Ku, Seoul 151-747 (KR)**
- **RYOO, Zae-Young**
  **Gunpo-City,**
  **Kyunggi-do 435-040 (KR)**
- **BAE, Euiyoung**
  **Seoul 137-040 (KR)**
- **LEE, Woo-Kyoung**
  **Seoul 151-021 (KR)**
- **CHO, Chul-Soo,**
  **No. B-503, Samho Garden Apartment**
  **Seoul 137-761 (KR)**
- **PARK, Sung-Hwan,**
  **Centre for Rheumatic Diseases**
  **D505,Banpo-Dong,Scocho-Ku, Seoul 137-701 (KR)**
- **KIM, Wan-Uk,**
  **No. 503-901, Mido 2nd Apartment**
  **Seoul 137-769 (KR)**

(74) Representative: **Bublak, Wolfgang et al**
**Bardehle, Pagenberg, Dost, Altenburg, Geissler,**
**Galileiplatz 1**
**81679 München (DE)**

(56) References cited:
**WO-A1-99/09956** **WO-A1-99/37315**
**WO-A2-99/02186** **US-A- 5 691 448**

- **Urata T. et al: (1999) J. Controlled Release 58: 133-141**
- **Desai M.P. et al: (1996) Pharm. Research 13: 1838-1845**
- **Uchida T. et al: (1997) Chem. Pharm. Bull. 45: 1539-1543**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> KIM, Ho-Youn
Park, Jong-Sang

<120> Immunological Tolerance-Induction Agent

<130> pct99081

<140> PCT/KR99/00000
<141> 1999-08-18

<160> 11

<170> PatentIn Ver. 2.0

<210> 1
<211> 20
<212> PRT
<213> Homo sapiens

<400> 1

```
Ala Arg Gly Phe Pro Gly Thr Pro Gly Pro Gly Gly Val Lys Gly His
 1               5                  10                  15

Arg Gly Tyr Pro
            20
```

<210> 2
<211> 20
<212> PRT
<213> Homo sapiens

<400> 2

```
Thr Gly Gly Lys Pro Gly Ile Ala Gly Phe Lys Gly Glu Gln Gly Pro
 1               5                  10                  15

Lys Gly Glu Pro
            20
```

<210> 3
<211> 20
<212> PRT
<213> Homo sapiens

<400> 3

```
Pro Gly Glu Arg Gly Leu Lys Gly His Arg Gly Phe Thr Gly Leu Gln
 1               5                   10                  15

Gly Leu Pro Gly
            20
```

<210> 4
<211> 16
<212> PRT
<213> Bovine

<400> 4

```
Cys Gly Glu Asx Gly Ile Ala Gly Phe Lys Gly Glu Gln Gly Pro Lys
 1               5                   10                  15
```

<210> 5
<211> 13
<212> PRT
<213> Homo sapiens

<400> 5

```
His Ser Leu Gly Lys Trp Leu Gly His Pro Asp Lys Phe
 1               5                   10
```

<210> 6
<211> 14
<212> PRT
<213> Homo sapiens

<400> 6

```
Asn Thr Trp Thr Thr Cys Gln Ser Ile Ala Phe Pro Ser Lys
 1               5                   10
```

<210> 7
<211> 16
<212> PRT
<213> Homo sapiens

<400> 7

```
Ile Ala Ala Thr Tyr Asn Phe Ala Val Leu Lys Leu Met Gly Arg Gly
 1               5                   10                  15
```

<210> 8

<211> 13
<212> PRT
<213> Homo sapiens

<400> 8

```
Val His Phe Phe Lys Asn Ile Val Thr Pro Arg Thr Pro
 1               5                   10
```

<210> 9
<211> 15
<212> PRT
<213> Homo sapiens

<400> 9

```
Asp Glu Gly Gly Tyr Thr Cys Phe Phe Arg Asp His Ser Tyr Gln
 1               5                   10                  15
```

<210> 10
<211> 20
<212> PRT
<213> Homo sapiens

<400> 10

```
Leu Gly Glu Leu Thr Ser Ser Glu Val Ala Thr Glu Val Pro Phe Arg
 1               5                   10                  15

Leu Met His Pro
            20
```

<210> 11
<211> 20
<212> PRT
<213> Homo sapiens

<400> 11

```
Ser Arg Leu Ser Lys Val Ala Pro Val Ile Lys Ala Arg Met Met Glu
 1               5                   10                  15

Thr Gly Thr Thr
            20
```

## Description

FIELD OF THE INVENTION

1. FIELD OF THE INVENTION

The present invention relates to an immunological tolerance-induction agent. More particularly, it relates to a use of biodegradable polymers as an immunological tolerance-induction agent and a method for treating autoimmune diseases in mammals.

2. DESCRIPTION OF THE PRIOR ARTS

Injection of a foreign substance (referred to as 'antigen') into a vertebrate organism may result in the induction of an immune response characterized by the production of specific antibodies capable of interacting with the antigen or the development of effector T lymphocytes and the precaution of soluble mediators at the site of encounter with the antigen. Although antibodies and T lymphocytes do play an essential role in protecting against hostile antigen, they can also participate in injurious processes, leading to destruction of host tissues. This is the case in autoimmune diseases where one's own antibodies and/or T lymphocytes react with antigen of one's own tissues and damage the tissues. This also is the case in allergic reactions characterized by an exaggerated immune response to certain environmental matters and which may result in inflammatory responses leading to tissue destruction.

An approach to treat effectively the diseases caused by unwanted or tissue damaging immunological reactions such as autoimmune diseases is to specifically suppress or decrease to an acceptable level the intensity of deleterious immune processes without affecting the remainder of the immune system. Immunological tolerance deals with all mechanisms that ensure an absence of destructive immune response to any given foreign substance.

Orally administered autoantigens suppress autoimmunity in animal models, including experimental allergic encephalomyelitis (EAE), collagen-induced arthritis (CIA), uveitis, and diabetes in the non-obese diabetic mouse (1-4). Oral tolerance describes the observation that a state of hyporesponsiveness follows an immunization with a previously and repetitively fed protein. Recently, the antigen-specific tolerance induction has been utilized as a treatment for various autoimmune diseases. For example, the repetitive administration of type II collagen (CII) or major basic protein (MBP; myeline basic protein) has been reported to be effective for the treatment of CIA or EAE, respectively. Likewise, oral administration of S-antigen can suppress the development of experimental uveitis (1-4). Oral tolerance induction has advantages that it doesn't elicit side effects including leukopenia, increased susceptibility of infection or malignance which can be induced by other drugs used currently for treating autoimmune disease, since it can selectively inhibit pathologic immune responses rather than non-specific immune suppression. However, irrespective of apparent ideality of oral tolerance induction, only few reports described the effectiveness of the oral tolerance in human disease, and even the results are not entirely uniform.

Several factors may affect the effectiveness of tolerance induction. Nature of antigen (soluble or particulate antigens) (5), antigen dose (6,7), genetic background (8), role of digestive flora (9), and antigen uptake (10) are known to be important factors in the determination of tolerance effect. In particular, oral tolerance is associated with the presence of immunologically relevant antigen in circulation. The amount of antigen absorbed intactly varies widely and is extremely small, ranging from 0.001% to 1% of the administered dose, which is partly attributed to the intraluminal digestion before the absorption of antigen (10-12). In this respect, the enhancement of the absorption rate of antigen administered orally seem to be promising in the tolerance therapy of human autoimmune disease.

Biodegradable microparticles have been studied as a drug carrier system, because of their advantages such as enhanced absorption, biocompatibility, sustained drug release and so on (13-15). Particularly, since particles can slowly release any entrapping substances into surroundings for a long period, they can deliver several kinds of drugs, hormones, and vaccines for 1 to 3 months, even though they are administered only once. Recently, it has been reported that particulate antigens administered orally are absorbed well into Peyer' s patches, which are the primary immune system of the gastrointestinal tract (16-18). Furthermore, soluble antigens entrapped by particulates can be protected from acidic and enzymatically hostile environments found at mucosal surfaces, facilitating uptake of the antigens by M cells overlying Peyer's patches and mucosal epithelium, thereby promoting antigen transport to local lymphoid follicles and associated lymph node, and possibly enabling the local accumulation of the antigen (19-22). Owing to these properties of particulate antigens, studies for particles are mainly focused on the vaccine delivery system in immunology.

Possible accumulation of particulate antigens in Peyer's patch and subsequent translocation to discrete anatomical compartments such as liver and spleen could modulate both mucosal and systemic immune responses. Recently, it is documented that several kinds of polymer have a potential to suppress pathologic immune response. Copolymer 1, a synthetic amino acid copolymer composed of L-alanine, L-glutamic acid, L-lysine, and L-tyrosine, has a beneficial effect on the development of EAE that is associated with down-regulation of T cell immune responses to MBP, an

autoantigen of EAE, and is mediated by Th2/3 type regulatory cells (23). In CIA, copolymer 1 also competitively inhibits the interaction of CII with RA-associated HLA-DR molecules and thereby suppresses CII reactive T cell responses (24). Polyethylene glycol (PEG), another straight amphophilic polymer, is also proved to prevent arthritis in CIA (25). To the contrary, it is reported that silicone polymer-grafted starch microparticles facilitate mucosal antigenicity although they does not retard the release of antigen or protect antigen from degradation, suggesting their advantageous use for oral vaccine delivery (26). Sum of the previous findings suggests that immunogenecity to particulate antigens administered orally may be different depending on the types and sizes of particles, *in vivo*. Moreover, the fate and role of particulate antigens in tolerance induction, if any, remain to be defined *in vivo.*

Rheumatoid arthritis (RA) is a representative autoimmune disease in human and characterized by destructive polyarthritis. Although the etiologic agent of RA remains unclear, CII is a strong candidate because of its abundance in cartilage, an immunologically privileged tissue, and because of its ability to induce destructive immune-mediate polyarthritis after immunization of susceptible rodents and higher primates (27,28). Lymphocytes responding to CII are identified in the peripheral blood, or in the joints of patients with RA (29), and antibodies to CII are also evident in the sera, synovial fluid (SF), and cartilage in patients with RA (30,31), suggesting CII may act as an autoantigen in human RA.

Antigen-specific immune suppression using CII has been tested in human RA. Even though orally administered CII shows an active suppression of arthritis in the animal model of RA, the oral tolerance using CII in human RA is not consistent between the experiments. Barnett *et al* have demonstrated that oral CII therapy is effective in most RA patients (32). In contrast, Sieper *et al* have reported that only a minority of patients responded to treatment with oral CII (33). Thus, it is unclear whether oral tolerance induction using CII could be an effective strategy in the treatment of human RA.

Controversial effect of oral tolerance therapy in human disease is also noted in other autoimmune diseases. This may be attributed to the insufficient antigen delivery into Peyer's patches in the intestine, main machinery of oral tolerance induction. Unfortunately, only very few attempts to overcome the intraluminal digestion and to enhance the rate and amount of antigen have been made in tolerance induction. Although the particles composed of polymer and protein have been proved for their usefulness for protein delivery, there has been no attempt to test or suggestion of the ability of PLGA particles or of active ingredients-entrapping PLGA particles to induce tolerance has been made.

Additional variable to determine the usefulness of tolerance therapy is easy availability of autoantigens including CII, MBP, S-antigens, and glutamic acid decarboxylase (GAD). Since these antigens should be purified from live animals to administer into human, they are expensive and have a risk of transferring some viruses contained in animals to human. In this respect, practical usefulness of these antigens is limited in treating human disease. Recently, it has been reported that the synthetic peptides encompassing immunodominant portions of afore-mentioned autoantigens, also can suppress the development of autoimmune disease, just like a whole molecule of antigen (34, 35, 36, 37 and 38).

WO 99/09956 discloses a composition for inducing or potentiating an immune response, preferably a CTL, T helper cell, or neutralizing antibody response in a subject, comprising an antigen and/or a non-antigen bioactive agent capable of inducing or potentiating such an immune response encapsulated in a polymeric composition, wherein the polymeric composition comprises a blend of (a) a polymer present in an amount sufficient to provide structural integrity to the polymeric composition, and (b) a rapidly biodegradable component, a rapidly dissolving component, a rapidly swelling component or a component that causes osmotic rupture of the encapsulated polymeric composition.

T. Urata et al [(Journal of Controlled Release 58 (1999), 133-141)] found that the addition of fatty acid esters enhanced the release rates of cyclosporine A from PLA (poly(L-lactic acid)).

T. Uchida [Chem. Phar. Bull. 45(9), 1539-1543 (1997)] et al disclose the production of PLA (polylactic acid) containing bovine insulin as a sparingly water soluble model drug using a water-in-oil-in-water (w/o/w) emulsion solvent evaporation method.

Synthetic peptides may be utilized to treat autoimmune diseases such as RA, multiple sclerosis, and uveitis, without the drawbacks associated with the difficulty in purifying autoantigens from animals. Tolerance induction using synthetic immunodominant peptides seems to be promising since these peptides may be prepared in an easy and economic way as well as they have no risk to transfer viral diseases.

The inventors conducted a series of oral tolerance induction using biodegradable polymer particles with or without an autoimmune antigen or its immunodominant peptide fragments entrapped therein in an animal experimental model of RA, a representative autoimmune disease, and tested the ability of polymer particles to induce tolerance and mechanisms thereof by immunologic assays and electron microscopy, and as result thereof, found out that the polymer particles can induce oral tolerance against autoimmune diseases and enhance oral tolerance of autoimmune antigens.

## SUMMARY OF THE INVENTION

The present invention relates to the use of particles of biodegradable polymers capable of reducing autoimmune response for preparing a medicament suitable for oral administration for treating autoimmune disease, wherein said biodegradable polymers are poly (DL-lactide-co-glycolide), polylactides or polyglycolides, wherein a single oral dose of said particles is suitable to be administered to induce tolerance against autoimmune response.

The present invention further relates to the use of particles of biodegradable polymers entrapping an autoimmune antigen for preparing a medicament suitable for oral administration for treating autoimmune diseases, wherein said particles of biodegradable polymers are capable of reducing autoimmune response, wherein said biodegradable polymers are poly (DL-lactide-co-glycolide), polylactides or polyglycolides, wherein a single oral dose of said particles is suitable to be administered to induce tolerance against autoimmune response.

The present invention further relates to the use of a composition for preparing a medicament suitable for oral administration for inducing tolerance for autoimmune diseases, which comprises as an active ingredient particles of biodegradable polymers entrapping an autoimmune antigen in an amount effective to induce tolerance against autoimmune response, and said particles of biodegradable polymers capable of reducing autoimmune response, wherein said particles have a diameter ranging from one hundred nanometer to several thousand nanometers, wherein said biodegradable polymers are poly (DL-lactide-co-glycolide), polylactides or polyglycolides, wherein a single oral dose of said particles is suitable to be administered to induce tolerance against autoimmune response.

The above and other objects, features and applications of the present invention will be apparent to those of ordinary skill by the following detailed description.

BRIEF DESCRIPTION OF THE DRAWINGS

Figure 1 is a SEM (Scanning Electron Microscopy) analysis depicting the PLGA particles entrapping type II collagen (PLGA-CII), in which the arrows indicate the diameter of a particle, approximating 300 nm;

Figure 2 is *in vitro* release of CII from PLGA particles, showing a slow release profile over 4 weeks and typical biphasic pattern;

Figure 3 (a) and 3 (b) are graphs depicting the severity and incidence of arthritis with the lapse of time in mice having a collagen-induced arthritis, respectively;

Figure 4 is a clinical assessment of arthritis in mice fed with PLGA-CII, native CII or acetic acid following subsequent challenges with CII and CFA. Data were expressed by mean arthritis index(Figure 4(a)), incidence of arthritic mice (Figure 4(b)), and percent arthritic limb(Figure 4(c)). In Figure 4(b) and 4(c), -•- denotes mice fed with PLGA-CII (40μg, once), -■- mice fed with CII (5μg, eight times), and -▲- mice fed with acetic acid (control). P value: * < 0.05, ** < 0.01, and *** < 0.001;

Figure 5 shows paw swelling of arthitic limbs in mice fed with CII or PLGA-II (left) and those fed with acetic acid (right);

Figure 6(a) and 6(b) show tolerogenic effect of PLGA entrapping an immunodominant peptide fragment of CII ('CII (255-274)');

Figure 7 shows tolerogenic effect of PLGA itself in term of clinical symptoms. Data were expressed by mean arthritis index(Figure 7(a)), incidence of arthritic mice(Figure 7(b) left), and percent arthritic limb(Figure 7(b) right).

Figure 8(a) shows the immunosuppressive effect of PLGA-CII or CII on IgG antibodies to CII and 8(b) shows the effect of PGLA-CII on T cell proliferative responses to CII;

Figure 9(a) and 9(b) are electron microscopic photographs for identifying PLGA particles in Peyer's patches, in which dendritic cell on day 14 and 35 after first feeding in Peyer's patch of mouse fed CII (5μg, eight times)(a) and dendritic cell in Peyer's patch of mouse fed PLGA-CII (40μg, once) on day 14 and 35 after feeding (b). In Figure 9, black arrows denote an intact from of PLGA particles, approximating 300nm in size, in the cytoplasm, and white arrow denotes a degraded from of PLGA particles in the lysozyme granule of dendritic cells; and

Figure 10 is a CII-specific immunohistochemical staining of Peyer's patches. Arrow indicates CII stained.

DETAILED EXPLANATION OF THE INVENTION

The present invention provides a use of biodegradable polymer particles, in particular polylactide-polyglycolide (PLGA) particles as an inducer of oral immunological tolerance, especially for autoimmune diseases.

The biodegradable polymers may contain an autoimmune antigen entrapped therein to enhance oral tolerance in mammals.

The term of 'autoimmune antigen' used herein means tissue-specific antigens that are themselves the subject of autoimmune attack and may include, among others, antigens ofEAE, CIA, uveitis, multiple sclerosis, insulin dependent diabetes mellitus (IDDM) and the like. In specifically, it include type II collagen, major basic protein (MBP), glutamic acid decarboxylase (GAD), S antigen and the like as well as immunodominant peptide fragments thereof. The immunodominant peptide fragments are known to those skilled in the art and may include the following SEQ. ID. NO. 1 through SEQ. ID. NO. 11 as well as their equivalents as long as they retain immunogenic property :

1) Rheumatoid arthritis (35)

CII (74-93); ARGFPGTPGLPGGVKGHRGYP (SEQ. ID. NO. 1)
CII (254-273); TGGKPGIAGFKGEQGPKGEP (SEQ. ID. NO. 2)
CII (924-943); PGERGLKGHRGFTGLQGLPG (SEQ. ID. NO. 3)
CII (255-270); CGEBGIAGFKGEQGPK (SEQ. ID. NO. 4)

2) Multiple sclerosis (36,37)

Myelin proteolipid protein (PLP) (139-151); HSLGKWLGHPDKF (SEQ. ID. NO. 5)
PLP (178-191); NTWTTCQSIAFPSK (SEQ. ID. NO. 6)
PLP (258-273); IAATYNFAVLKLMGRG (SEQ. ID. NO. 7)
Major basic protein (87-99); VHFFKNIVTPRTP (SEQ. ID. NO. 8)
Myelin oligodendrocyte glycoprotein (92-106); DEGGYTCFFRDHSYQ (SEQ. ID. NO. 9)

3) Autoimmune Uveitis (38)

S antigen (343-362); LGELTSSEVATEVPFRLMHP (SEQ. ID. NO. 10)

4) IDDM (42)

Glutamic acid decarboxylase-65 (524-543); SRLSKVAPVIKARMMEYGTT (SEQ. ID. NO. 11)

The sequence of the immunodominant peptide fragments may be slightly varied depending on their origins, and the present invention encompasses the use of polymer particles entrapping the peptide fragments regardless of their origin as long as they show the tolerance inducing property in living body. Both of synthetic and naturally occurring peptide fragments may be employed according to the present invention, and synthetic peptide fragments are advantageously used due to easy and unexpensive production thereof.

The term of 'autoimmune disease' used herein means a condition where the immune system mistakenly perceives the body's own tissue as foreign and mounts an abnormal immune response against it and may include experimental allergic encephalomyelitis (EAE), collagen induced arthritis (CIA), rheumatoid arthritis (RA), uveitis, multiple sclerosis, IDDM, non-obese diabetes, and the like. Rheumatoid arthritis can be advantageously treated by the present invention. In particular, the polymer particles without antigen can advantageously be used to treat Th1-mediated or T cell-mediated autoimmune diseases such as rheumatoid arthritis, IDDM, uveitis, multiple sclerosis, autoimmune thyroiditis (e.g., Grave's disease, Hashimoto's thyroiditis), autoimmune hepatitis, interstitial pneumonitis or glomerulonephritis, and their corresponding diseases in animal models.

Throughout the application, the term of 'polymers' or 'PLGA', or the part of 'PLGA' in the terms of 'PLGA-CII' (or 'PLGA entrapping CII'), or 'PLGA-CII(amino acid number)' should be understood to indicate particles made therefrom having the desired size unless they are intentioanlly used to stand for the compound itself. Moreover, the term of 'particle (s)' is used to indicated to include both the polymer particles and the antigen-entrapping polymer particles, unless indicated otherwise.

The autoimmune antigen which will be entrapped by biodegradable polymers may be selected by those skilled in the art without difficulty depending on the kind of the autoimmune diseases to be treated. For example, CII or its immunodominant peptides will be entrapped for treating RA in human and collagen-induced arthritis in animal models (rat, mouse, monkey, etc); major basic protein and its immunodominant peptide for multiple sclerosis in human and experimental autoimmune encephalitis in animal models (rat, mouse, etc); GAD for IDDM in human and experimental diabetes mellitus in animal models (rat, mouse, etc); and S antigen for autoimmune uveitis in human and autoimmune uveitis in animal models (rat, mouse, etc).

The mammals to which the present invention can be applied may include, but not limited to, rat, mouse, monkey, various experimental animal models, and human.

The biodegradable and biocompatible polymers which may be employed according to the present invention are selected from poly(DL-lactide), polyglycolic acid and poly(DL-lactide-co-glycolide)('PLGA'). The PLGA is preferred for use in accordance with the present invention since its safety in living body is well established. Polymeric materials for preparation of the particles are available commercially. These biodegradable polymers degrade through hydrolysis of polymer linkages to yield biocompatible products such as lactic acid and glycolic acid for PLGA, upon exposure to water.

The term of 'PLGA' is used herein to refer to the various copolymers of lactic and glycolic acids, which can be represented by following general formula:

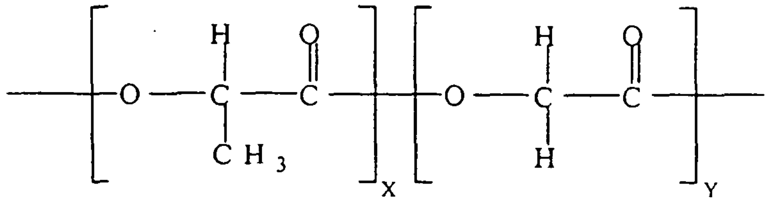

PLGA 50:50 (X : Y = 50 : 50)

The molecular weight of the polymers ranges from about 25,000 Daltons to about 120,000 Daltons. The ratio of lactide units to glycolide units ranges from 100:0 to 50:50. For example, PLGA having a ratio of lactide : glycolide = 85:15, which can be obtained from Birmingham Polymers, Incorporate, has an inherent viscosity of 0.58 dL/g and a molecular weight of approximately 90,900 daltons. It has been reported to have a biodegradation time up to approximately 10 months, depending on surface area, porosity and molecular weight. On the other hand, 50:50 PLGA polymer has a reported degradation time of 2 months.

The particles of biodegradable polymers (hereinafter, referred sometimes as simply 'particles') have a diameter ranging from one hundred nanometer to several thousand nanometers, preferably less than about 500nm, and more preferably about 200 to about 300nm. The particles may be prepared by conventional method, for example, by dispersing an organic solution of the polymer into an aqueous solution and then evaporating the solvent.

The particles entrapping an autoimmune antigen may be prepared by conventional water-in-oil-in water emulsion solvent evaporation methods. For example, the biologically active ingredients in a suitable aqueous solvent is emulsified with a solution of biodegradable polymers in an organic solvent such as methylene chloride to form an oil-in-water (o/w) emulsion. Then the o/w emulsion is emulsified with an aqueous solution of water-soluble polymer (e.g. polyvinyl alcohol) to give a w/o/w emulsion. Then the resulting w/o/w emulsion is evaporated to remove the organic solvent. When immunodominant peptide fragments are used as an autoimmune antigen, they may advantageously be coupled in advance to a proper polymer such as PEG (MW more than 2,000) or polypeptides in order to moderate their release rate from the particles. PEG may be advantageously used for this purpose.

The particles have preferably a size of less than 500nm, and more preferably between 200nm and 300nm. The loading of antigen varies between about 0.5% and about 10%. Particles with desired size were obtained by optimizing various formulation parameters such as volume and concentration of each solution used, a kind of surfactant materials, and time period of energy input, energy sources such as sonicator, homogenizer or vortex used to form the water/oil/water emulsions.

The particles withour or with an antigen entrapped therein may be administered orally to mammals such as human, cats, dogs, rats, mice, monkeys and the like for the purpose of treating autoimmune diseases.

The dose of the particles for oral administration may vary and be determined by those skilled in the art depending on the kind and severity of the diseases and condition of the subjects. For example, to treat RA, the dose may be within the range 0.1 - 1000mg of PLGA/kg of weight, and preferably 1 - 100 mg of PLGA/kg of weight, and more preferably 10 - 40mg of PLGA/kg of weight.

When the particle entrapping CII or its immunogenic peptide fragments is used, the dose of antigen may be within the range of 1 - 10000μg, preferably 10 - 1000 μg, and more preferably 20 - 160μg.

The particles without or with an entrapped antigen may be administered orally and only single administration thereof can give a significant and satisfactory suppression of autoimmune response in mammals.

## Examples

The present invention will be described in more detail by way of various Examples, which should not be construed to limit the scope of the present invention.

MATERIALS AND METHODS

**Animal**

DBA1 mice obtained from Jackson Laboratories (Bar Harbor, ME) were maintained in groups of three to five in polycarbonate cage and fed standard mouse chow and water adlibitum. The environment was specific pathogen-free for the mice obtained from Jackson Laboratories. Neonatal mice were obtained by breeding mice in inventor's facility. Mice were immunized at 8-12 weeks as described previously (25, 27, 28).

**Preparation of type II collagen**

Intact bovine CII was extracted and purified from articular cartilage of calf as described previously (39). Following steps were carried out; solubilization with pepsin from cartilage slices after removal of proteoglycans by exposure to neutral 4 M guanidine HCl, repeated precipitation from 0.5 M acetic acid at 0.86 M NaCl, and dialysis against 0.02 M $Na_2HPO_4^-$; removal of pepsin and proteoglycans by DEAE chromatography; and removal of type I collagen by differential salt precipitation at neutral pH. Purity was confirmed by SDS-PAGE. Lyophilized native bovine CII was dissolved in 0.1N acetic acid at 1mg/ml, dialyzed against 50 mM Tris, 0.2M NaCl, and then sterilized by filtering through a 0.2 μm micropore filter.

**Preparation of PLGA entrapping CII**

Entrapping of CII within PLGA was performed as described previously (40). A solution of bovine CII (10 % w/v) in 0.5 M acetic acid (internal aqueous phase) was initially emulsified with a solution of PLGA (3 % w/v) in methylene chloride (oil phase), using a probe sonicator to form a water-in-oil (w/o) emulsion. The resulting emulsion was added to a 5% (w/v) PVA solution (external water phase) followed by second emulsification, resulting in a water-in-oil-in water (w/o/w) emulsion. The w/o/w emulsion was stirred over a magnetic stir plate overnight at room temperature to evaporate methylene chloride. Upon evaporation the volatile organic solvent was removed, resulting in an aqueous suspension of particles. Particles with proper size were then recovered by ultracentrifugation. The particles were washed twice with water and resuspended in water prior to lyophilization. The particles are sometimes referred to as 'PLGA-CII' for simplicity. Figure 1 is an electroscopic picture of the PLGA particles having a size of about 300nm.

**Peptide synthesis and entrapment of peptide with PLGA.**

Immunodominant peptide fragment of bovine CII (255-270) : CGEBGIAGFKGEQGPK (SEQ. ID. NO. 4) was synthesized and then entrapped with PLGA by organic solvent evaporation methods (40). Synthetic peptide was conjugated with polyethylene glycol (PEG, MW5000), because this peptide was short and not soluble in methylene chloride which was an organic solvent used in making particle. Conjugation of peptide with PEG (methoxy-PEG-NH2) was as follows. The linker of SPDP (N-Succinimidyl 3-[2-pyridyldithio] propionate) was used in conjugation of peptide with PEG. 30mg of PEG was dissolved in 0.2ml of phosphate buffer (20mM, pH 7,5) and 5mg of SPDP in 0.2ml of DMSO. The two solutions were mixed and incubated at room temperature for 1 hour. After the reaction, SPDP-conjugaed PEG was separated by gel filtration column chromatography eluted with distilled water at the flow rate of 0.5 ml/min, followed by freeze-drying the separated fractions. The resulted PEG-SPDP was dissolved with peptide (10mg) in 0.5ml of phosphate buffer (20mM, pH 7,5), and incubated at room temperature overnight. The PEG-conjugated peptide was applied to the same gel filtration chromatography eluted with distilled water at the same flow rate (0.5ml/min). The purified fractions were freeze-dried and stored at -20 °C.

PLGA entrapping peptide was prepared as the same method as described for the preparation of PLGA-CII. The solution of PEG-peptide(2.5mg) and PLGA (50mg) in methlylene chloride (1.5ml) was emulsified with a solution of 5% PVA, using a probe sonicator for 20 seconds. The emulsion was stirred over a magnetic stir plate for 5 hours at room temperature to evaporate the organic solvent. After the evaporation, the suspension was centrifuged at 500 × g for 5 min to remove the particles. The resulting particles were washed twice by ultracentrifuging and resuspending, and lyophilized. The particles are sometimes referred to as 'PLGA-CII(255-270)' for the simplicity.

**Induction of CIA and clinical evaluation of arthritis**

Mice were immunized with native CII at 8 to 12 weeks of age. CII dissolved in 0.1 N acetic acid (2mg/ml) was emulsified (1:1 ratio) with CFA (Complete Freund Adjuvant) at 4°C. Mice were received 0.1ml of the emulsion containing 100 μg CII into base of tail as a primary immunization. Booster injection(50 μg CII) was given into footpad on day 14. Mice were observed two to three times per week for onset, duration, and severity of joint inflammation over a period of

12 weeks after primary immunization. Each limb was assessed on a 0- to 4-point scale by the following criteria: 0 = normal; 1 = mild inflammation of a single area (i.e., either the midfoot, ankle, or toes); 2 = moderately severe arthritis involving toes and ankle or midfoot; 3 = severe arthritis involving entire paw; and 4 = severe arthritis resulting in ankylosis and loss of joint movement. Since the left foot that was used for booster immunization was excluded from the evaluation, the possible maximum arthritis score was 12. Mean arthritic index, incidence of arthritis (%), and percent arthritic limbs were used for comparison and calculated as follows:

$$\text{Mean arthritis index} = \text{Sum of the arthritic score / Total number of mice}$$

$$\text{Incidence of arthritis (\%)} = \frac{\text{Number of mice with arthritic score} > 2 \text{ in any limb}}{\text{Total number of mice}} \times 100$$

$$\text{Percent arthritic limbs (\%)} = \frac{\text{Number of limbs with arthritic score} > 2}{\text{Total number of limbs}} \times 100$$

**Tolerance induction**

Mice were fed 8 times with 5μg CII on days -17, -15, -13, -11, -9, -7, -5, -3 relative to the primary immunization with CII in CFA on day 0. Control mice were fed 8 times with 0.1 N acetic acid. PLGA, PLGA-CII 40μg (cumulative dose of CII in mice repetitively fed with CII), or PLGA-CII(255-270) 4μg were fed only once on day -17.

**Assessment of T cell proliferation**

Draining or mesenteric lymph node, and spleen were removed from animal at 14 days after immunization and dissociated and washed in RPMI 1640. Lymph nodes and spleen were individually minced into single cell suspensions in RPMI 1640 and washed three times. Cells (5 X $10^5$/well) were cultured in 96-well microtiter plates (Nunc, Roskilde, Denmark) with or without 100 μg/ml of CII in 0.3 ml of Click's medium supplemented with 0.5% mouse serum at 37°C, 5% humidified $CO_2$ for 4 days. Eighteen hours before the termination of the cultures, 1 μCi [$^3$H] thymidine was added to each well. Cells were harvested onto glass fiber filters and counted on a Matrix 96 direct ionization β-counter (Packard Instrument Co, Meridan, CT). The results are presented as stimulation indices (SI), calculated as the ratio of cpm in the presence of antigens / cpm without antigens.

**Assay for IgG antibodies to bovine native type II collagen**

Sera were collected on day 28 after primary immunization and stored at -20°C until the assay. The levels of IgG anti-CII in sera were determined by enzyme-linked immunosorbent assays kit (Chondrex, Redmond, WA). The optical density of standard serum which was serially diluted 2-fold was expressed as 100, 50, 25, 12.5, 6.25, 3.125 arbitray units (a.u.), respectively, according to the manufacture's instructions. The relationship of the optical density measured in standard serum diluted serially and arbitrary unit showed a good linear correlation in all determinations (r>0.98, data not shown). The concentrations of IgG anti-CII in the sera diluted 1: 4000 are presented as relative values (a.u.) compared to the optical density of the standard serum which was diluted serially.

**Immunohistochemical staining of CII in Peyer's patch**

To determine whether CII entrapped within PLGA is still present in the intestine for a long period, immunohistochemical staining of CII was performed in Peyer's patches. Briefly, Peyer's patches were obtained from mice administered orally PLGA entrapping CII on days 14 and 35 after feeding. Peyer's patches from mice fed with repetitive CII or acetic acid was used for control tissue. After fixing with periodate-lysin paraformaldehyde solution at 4°C overnight, tissues were dehydrated with alcohol. After washing, wax (polyethylene glycol 400 distearate) embedding and microsection (5 μm) of tissue were done. To remove peroxidase activity within tissue, methanolic $H_2O_2$ was added and then tissues were blocked with normal sera for 1 hour. After tissue were incubated overnight with goat monoclonal antibodies to bovine CII, generous gifts from Dr. Andrew H Kang, DAG (peroxidase cojugated $F(ab')_2$, Donkey anti-goat IgG, Jackson Immunoresearch Laboratories) was added to tissue. Biotinylated antibody was then added to tissues and color reaction was detected with diamino-benzidine (DAB) on microscopy.

***In vitro* release of entrapped antigen from PLGA**

The *in vitro* release of CII from PLGA particles was evaluated for 4 weeks under physiologic conditions at 37°C. FITC-labeled collagen was used in *in vitro* release test to increase sensitivity. After labeling collagen with FITC, the particles were prepared as described above. Then, the particles were dispersed in phosphate buffer (PH 7.4, 0.1M) and maintained at 37°C with mild stirring. Keeping the sample in this condition, an aliquot of the sample was taken and its fluorescence was measured by spectrofluorometer periodically.

**Characterization of particles by electron microscopy**

Scanning electron microscopy (SEM) was performed to determine the surface topography of the particles. Particles were gold coated using a sputter gold coater (Denton Vacuum Inc, Cherry Hill, NJ) at 40 miliampere current and 50 militorr pressure for 200 seconds to the thickness of 300 A°. Samples of gold coated particles were cooled over dry ice prior to SEM observations to avoid their melting under magnification due to electron beam exposure (Hitachi S570, Chery Hill, NJ). To characterize PLGA particles *in vivo* and to determine whether PLGA particles are still present in the intestine for a long period, a ultra-thin frozen section of Peyer's patches was obtained on days 14 and 35 after feeding of PLGA-CII and then the presence of PLGA particles was investigated on electron microscopy.

**Statistics**

Comparisons of numerical data between groups were performed by the student T-test and ANOVA and for categorical data by a chi-square test or Fisher's exact probability test. P values less than 0.05 were considered significant.

<u>RESULTS</u>

**1. Characterization of PLGA particles**

Particles with desired size were obtained by optimizing various formulation parameters and energy source input used to form the water/oil/water emulsions. As can seen from the SEM analysis, nanoparicles were spherical with smooth uniform surfaces with more than 80% particles in the desired size range. Discrete spherical particles had a mean diameter of 299.7 ± 4.9 nm (Figure 1). To find out collagen loading of particles, weighed particles were dissolved in 0.4 N NaOH, 10 % SDS (w/v) solution. And then, the solution was assayed for collagen by Lowry method. 1.2 % of total weight was composed of collagen. Encapsulation efficiency was approximately 6.5%.

This study demonstrated that PLGA particles produced *in vitro* an initial burst release of 10 to 20% of encapsulated CII within two days. The subsequent release of CII up to 20 days was at a slower releasing rate during which 10% to 20% of CII was released. The slow release phase was followed by a second burst release from day 15 to 30 possibly due to hydrophobic erosion of the polymer matrix (Figure 2).

**2. Establishment of collagen induced arthritis**

Figure 3(a) and 3(b) shows the incidence and severity of collagen-induced arthritis with the laspe of time in an animal model, respectively.

As shown in Figure 4(b) and 4(c), in mice (n=9) fed acetic acid and injected with CII and CFA, CIA developed from 3 weeks. The severity and incidence of arthritis peaked at 5 weeks, reaching 5.1 of mean arthritic index (maximum 12) and 100% of incidence, respectively and were decreased over time thereafter, which is consistent with previous

reports (25, 27, 28).

### 3. Tolerance induction with PLGA entrapping CII

To test the ability of single administration of PLGA-CII to suppress CIA, the mean arthritis index, incidence, and number of arthritic limbs were compared between mice (n=8) fed with PLGA-CII once and those fed with eight times of CII (n=8) or acetic acid (n=8), at five weeks after primary immunization. As shown in Figure 5, mice fed with PLGA-CII and mice fed with CII had significant lower levels of the mean arthritis index (PLGA-CII 0.9±0.3, CII 2.8±0.9, acetic acid 4.5±1.1, p<0.001), incidence (PLGA-CII 0%, CII 50%, acetic acid 100%, p<0.001), and number of arthritic limbs (PLGA-CII 0/24 [0%], CII 5/24 [20.8%], acetic acid 14/24 [58.3%], p<0.001) compared to those fed with acetic acid. Comparison of clinical parameters between PLGA-CII and CII fed mice showed that mice fed with PLGA-CII had significant lower levels of the mean arthritis index (p<0.001), incidence (p=0.02), and number of arthritic limbs (p=0.008) compared to those fed with CII. These observation demonstrated that the single administration of PLGA-CII successfully suppressed the incidence of and relieved the severity of arthritis, and even more effective to induce tolerance compared to repetitive administration with same cumulative dose of CII. Figure 5 showed a remarkable decrease of paw swelling in tolerized mice fed CII or PLGA-CII compared to acetic acid fed mice.

### 4. Tolerance induction using PLGA entrapping CII (255-274).

The mean arthritis index, incidence, and number of arthritic limbs were lower in mice (n=4) fed with PLGA-CII (255-274) compared to mice (n=4) fed with acetic acid, at five weeks after primary immunization. Mean arthritis index; 3.5(0.6 versus 1.0(0.8, p=0.002, incidence; 25% versus 100%, number of arthritic limbs 1/12 [8.3%] versus 4/12 [33.3%] (Figure 6). These data strongly suggest that PLGA-CII (255-270) may be useful to treat RA. In addition, a single administration of PLGA entrapping synthetic peptides could be applicable to treat other autoimmune diseases such as multiple sclerosis, autoimmune uveitis, and insulin dependent diabetes mellitus (IDDM) in which repetitive oral administration of synthetic immunodominant peptides is known to be effective to suppress disease severity. In this context, representative synthetic peptides containing immunodominant portions of autoantigen, which can be entrapped in PLGA, are listed in the above.

### 5. Assessment of the ability of PLGA to induce tolerance

In addition to the slow release property of entrapped protein, several kinds of particulate antigen are known to have a potential to modulate immunogenecity by itself. To assess the ability of PLGA itself to induce tolerance, the inventors conducted a series of CIA induction in mice fed with PLGA. As a result, mice fed once with PLGA (n=8) had significant lower levels of mean arthritis index and percent arthritic limbs compared to mice fed with acetic acid (n=7) when assessed at 5 weeks after primary immunization (mean arthritic index; 1.8±0.8 versus 3.9±1.9, p=0.019, percent arthritis limb; 10/21 [12.9%] versus 3/24 [42.9%], p=0.01) (Figure 7(a)).

The incidence of arthritis also tended to be lower in mice fed with PLGA than those fed with acetic acid (p=NS). Comparison of clinical parameters at 5 weeks between PLGA and PLGA-CII administered mice showed that the incidence of arthritis was significantly lower in mice fed with PLGA-CII than PLGA alone (0% versus 50%, p=0.03). Mean arthritis index and percent arthritic limb tended to be lower in PLGA-CII fed mice, but they did not reach statistical significance (Figure .7(b)). These data demonstrated that PLGA alone could suppress CIA, but its potency was more enhanced by CII entrapping. These observations are strong evidences that tolerogenecity by PLGA-CII is achieved by the mechanism of immunosuppressive effect of PLGA itself (tolerance inducing effect) as well as repetitive stimulation of intestinal immune cells by PLGA-CII as a carrier system (tolerance enhancing effect).

### 6. Effect of PLGA-CII on autoimmune response to CII

Circulating IgG antibodies to CII were significantly lower in CII-fed (n=9) and PLGA-CII-fed mice (n=9) compared to acetic acid-fed mice (n=9) (PLGA-CII; 6.4±7.7, CII; 15.2±18.9, acetic acid; 1.8±0.8, p<0.01). PLGA-fed mice tended to have lower levels of IgG antibodies to CII compared to CII-fed mice (p=NS). Assay for T cell proliferative responses to CII in draining or mesenteric lymph node and spleen also showed that mice fed with PLGA-CII, PLGA and CII had lower levels of T cell proliferative responses to CII (Figure 8(a) and 8(b)). These observations clearly showed that PLGA-CII or PLGA itself could suppress both humoral and cellular autoimmune responses systemically when administered orally, which constructs an important mechanism of PLGA to induce tolerance since both T cells responding to CII and IgG antibodies to CII, are known to play a critical role in arthritis induction of susceptible animals.

### 7. Identification of PLGA particles in Peyer's patches

To characterize PLGA particles *in vivo* and to determine whether PLGA particles are still present in the intestine for a long period, the presence of PLGA particles was investigated in Peyer's patches on days 14 and 35 after feeding of PLGA, which is the time that any soluble antigen can not be detected in the intestine, by electron microscopy. As shown in Figure 9(b), PLGA particles having asize of about 300 nm were found in dendritic cells of Peyer's patches in an aggregated manner. They were located on the cytoplasm in an intact form as well as on lysozyme granules in a degraded form. Surprisingly, dendritic cells phagocyting PLGA particles revealed an increased amount of lysozyme granules, indicating cellular activation.

However, these findings were not detected in Peyer's patches from mice fed CII or acetic acid, repetitively (Figure 9(a)). Recently, it is reported that hyperplasia or activation of dendritic cells plays a pivotal role in tolerance induction, in contrast with the generally accepted view of dendritic cells as immunostimulatory antigen presenting cells. Our data clearly showed that, like results from *in vitro* release test, PLGA particles were present in dendritic cells of Peyer's patches with a slow degradation, at least up to 35 days and could activate dendritic cells, which may explain how PLGA particles can suppress the systemic autoimmune response to CII as well as the incidence and severity of arthritis. This may be one of the possible mechanism that PLGA particles contribute to tolerance induction (tolerance induction effect).

### 8. Persistence of CII in Peyer's patches from mice single fed PLGA-CII

On CII specific immunohistochemical staining of Peyer's patches, we found that CII was densely stained arround dendrtic cells from PLGA-CII fed mice on days 14 and 35, which is the time that any soluble antigen can not be normally detected in the intestine, which is consistent with the findings in *in vitro* release test (Result 1, Figure 3). However, these findings were not noted in mice fed with CII or acetic acid, repetitively (Figure 10). These findings demonstrated that CII was protected from acids and enzymatically hostile environments and released slowly from PLGA. This is also persuasive evidence that PLGA-CII contributes to tolerance induction in a manner of chronic antigenic stimulation of intestinal immune cells, driven by long-lasting CII.

<u>References</u>

1.Weiner HL, Mackin GA, Matsui M, et al: Double-blind pilot trial of oral tolerization with myelin antigens in multiple sclerosis. Science 259:1321-1324, 1993.

2. Trentham DE, Dynesius-Trentham RA, Orav EJ, et al: Effects of oral administration of type II collagen on rheumatoid arthritis. Science 261:1727-1730, 1993.

3. Zhang ZJ, Davidson L, Eisenbarth G, et al: Suppression of diabetes in non-obese diabetic mice by oral administration of porcine insulin. Proc Natl Acad Sci USA 88:10252-10256, 1991.

4. Nussenblatt RB, Caspi RR, Mahdi R, et al: Inhibition of S-antigen induced experimental autoimmune uveoretinitis by oral induction of tolerance with S-antigen. J Immunol 144:1689-1695, 1990.

5. Sun JB, Holmgren J, Czerkinsky C : Cholera toxin B subunit: an efficient transmucosal carrier-delivery system for induction of peripheral immunological tolerance. Proc Natl Acad Sci USA 91:10795-10799, 1994.

6. Melamed D, Friedman A: In vivo tolerization of Th1 lymphocytes following a single feeding with ovalbumin: anergy in the absence of suppression. Eur J Immunol 24:1974-1981, 1994.

7. Melamed D, Fishman-Lovell J, Uni Z, et al: Peripheral tolerance of Th2 lymphocytes induced by continuous feeding of ovalbumin. Int Immunol 8:717-724, 1996.

8. Stokes CR, Swarbrick ET, Soothill JF: Genetic differences in immune exclusion and partial tolerance to ingested antigens. Clin Exp Immunol 52:678-684, 1983.

9. Moreau MC, Coste M, Gaboriau V, et al: Oral tolerance to ovalbumin in mice: effect of some parameters on the induction and persistence of the suppression of systemic IgE and IgG antibody responses. Adv Exp Med Biol 371: 1229-1234, 1995.

10. Peng HJ, Chang ZN, Han SH, et al: Chemical denaturation of ovalbumin abrogates the induction of oral tolerance of specific IgG antibody and DTH responses in mice. Scand J Immunol 42:297-304, 1995.

11. Peng HJ, Turner MW, Strobel S: The generation of a 'tolerogen' after the ingestion of ovalbumin is time-dependent and unrelated to serum levels of immunoreactive antigen. Clin Exp Immunol 81:510-515, 1990.

12. Hanson LA, Telemo E, Wiedermann U, et al : Sensitization and development of tolerance via the gut. Pediatr Allergy Immunol 4:16-20, 1993.

13. Couvreur P, Puisieux F: Nano- and microparticles for the delivery of polypeptides and proteins: Adv. Drug Del.

Rev 10:141, 1993.
14. Lavelle EC, Sharif S, Thomas NW, et al: The importance of gastrointestinal uptake of particles in the design of oral delivery systems. Adv. Drug Del. Rev 18:5, 1995.
15. Andrianov AK, Payne LG: Polymeric carriers for oral uptake of microparticulates: Adv. Drug Del. Rev 34:155, 1998.
16. Florence AT: The oral absorption of micro- and nanoparticulates: neither exceptional nor unusual: Pharm. Res 14:259, 1997.
17. Jani P, Halbert GW, Langridge J, et al: Particle Uptake by the Rat Gastrointestinal Mucosa: Quntitation and Particle Size Dependency: J. Pharm. Pharmacol 42: 821, 1990.
18. Jani P, Florence AT, McCarthy DE: Further Histological evidence of the gastrointestinal absorption of polystyrene nanospheres in the rat: Int. J. Pharm 84:245, 1992.
19. Jepson MA, Clark MA, Foster N et al: Targeting to intestinal M cells. J. Anat.189: 507-516, 1996.
20. Moldoveanu Z, Novak M, Huang W-Q et al: Oral immunization with influenza in biodegradable microspheres. J. Infect. Dis 167: 84-90, 1993.
21. Smith MW, Thomas NW, Kenkins PG, et al: Selective transport of microparticles across Peyer's patch follicle-associated M cells from mice and rats. Exp Physiol. 80: 735-743, 1995.
22. Krey A, Giannasca KT, Weltzein R et al. Role of the glycocolyx in regulating access of microparticles to apical plasma membranes of intestinal epithelial cells: Implications for microbial attachment and oral vaccine targeting. J. Exp. Med 184:1045-1059, 1996.
23. Teitelbaum D, Arnon R, Sela M: Immunomodulation of experimental autoimmune encephalitis by oral administration of copolymer 1. Proc Natl Acad Sci USA 96:3842-3847, 1999.
24. Fridkis-Hareli M, Rosnoleic E, Fugger L, et al: Synthetic amino acid copolymers that bind to HLA-DR proteins and inhibit type II collagen-reactive T cell clones.
25. Ito HO, So T, Ueda T, et al: Prevention of collagen induced arthritis (CIA) by treatment with polyethylene glycol-conjugated type II collagen; distinct tolerogenic property of the conjugated collagen from the native one. Clin Exp Immunol 108;213-219, 1997.
26. Heritage PL, Loomes LM, Jianxiong J, et al. Novel polymer-grafted starch microparticles for mucosal delivery of vaccines. Immunology. 88:162-168, 1996.
27. Trentham DE, Townes AS, Kang AH: Autoimmunity to type II collagen: An experimental model of arthritis. J Exp Med 146:857-868, 1977.
28. Courtenay JS, Dallman MJ, et al: Immunisation against heterologous type II collagen induces arthritis in mice. Nature 283:666-668,1980.
29. Trentham DE, Dynesius RA, et al: Cellular sensitivity to collagen in rheumatoid arthritis. N Eng J Med ;299: 327-332, 1978.
30. Rudolphi U, Rzepka R, Batsford S, et al: The B cell repertoire of patients with rheumatoid arthritis. Increased frequencies of IgG+ and IgA+ B cells specific for mycobacterial heat-shock protein 60 or human type II collagen in synovial fluid and tissue. Arthritis Rheum 40:1409-1419, 1997.
31. Tarkowski A, Klareskog L, Carlsten H, et al: Secretion of antibodies to types I and II collagen by synovial tissue cells in patients with rheumatoid arthritis. Arthritis Rheum 32:1087-1092, 1989.
32. Barnett ML, Kremer JM, Clair EW, et al: Treatment of rheumatoid arthritis with oral type II collagen : Results of a multicenter, double-blind, placebo-controlled trial. Arthritis Rheum 41:290-297, 1998.
33. Sieper J, Kary S, Sorensen H, et al: Oral type II collagen treatment in early rheumatoid arthritis : a double-blind, placebo-controlled, randomized trial. Arthritis Rheum 39:41-51, 1996.
34. Khare SD. Krco CJ. Griffiths MM, et al: Oral administration of an immunodominant human collagen peptide modulates collagen-induced arthritis. J Immunol 155:3653-3659, 1995.
35. Krco CJ, Pawelski J, Harders J, et al: Characterization of the antigenic structure of human type II collagen. J Immunol:156:2761-2768, 1996.
36. Yu M, Johnson JM, Tuohy VK: A predictable sequential determinant spreading cascade invariably accompanies progression of experimental autoimmune encephalomyelitis: a basis for peptide specific therapy after onset of clinical disease. J Exp Med 183:1777-1788, 1996.
37. Tuohy VK, Yu M, L, et al: Spontaneous regression of primary autoreactivity during chronic progression of experimental autoimmune encephalomyelitis and multiple sclerosis. J Exp Med 189:1033-42, 1999.
38. Vrabec TR, Gregerson DS, Dua HS, et al: Inhibition of experimental autoimmune uveoretinitis by oral administration of S-antigen and synthetic peptides. Autoimmunity 12:175-84, 1992.
39. Stuart JM, Cremer MA, Kang AH, et al: Collagen induced arthritis in rats: evaluation of early immunologic events. Arthrits Rhem 22:1344, 1978.
40. Desai MP, Labhasetwar V, Amidon GL, et al: Gastrointestinal Uptake of Biodegradable Microparticles: Effect of Particle Size. Pharm. Res 13: 1838, 1996.
41. Kakimoto K, Katsuki M, Hirofujii T, et al: Isolation of T cell line capable of protecting mice against collagen

induced arthritis in mice. J Immunol 140:78-83, 1988.
42. Chen SL, Whiteley PJ, Freed Dc, et al: Responses of NOD congenic mice to a glutamic acid decarboxylase-derived peptide. J Autoimmunity. 7:635-41, 1994.

## Claims

1. Use of particles of biodegradable polymers capable of reducing auto-immune response for preparing a medicament suitable for oral administration for treating autoimmune diseases, wherein said biodegradable polymers are poly

(DL-lactide-co-glycolide), polylactides or polyglycolides, wherein a single oral dose of said particles is suitable to be administered to induce tolerance against autoimmune response.

2. The use according to Claim 1, wherein said autoimmune diseases are one of Th1-mediated or T cell-mediated autoimmune diseases selected from the group consisting of rheumatoid arthritis, insulin dependent diabetes mellitus, uveitis, multiple sclerosis, autoimmune thyroiditis, autoimmune hepatitis, interstitial pneumonitis and glomerulonephritis, and their corresponding diseases in animal models.

3. The use according to Claim 2, wherein said autoimmune disease is rheumatoid arthritis.

4. The use according to any one of Claim 1 to Claim 3, wherein said medicament is suitable for administration to human, rats, mice and monkeys.

5. The use according to any one of Claim 1 to Claim 3, wherein said particles have a size of less than about 500nm.

6. Use of particles of biodegradable polymers entrapping an autoimmune antigen for preparing a medicament suitable for oral administration for treating autoimmune diseases, wherein said particles of biodegradable polymers are capable of reducing autoimmune response, wherein said biodegradable polymers are poly (DL-lactide-co-glycolide), polylactides or polyglycolides, wherein a single oral dose of said particles is suitable to be administered to induce tolerance against autoimmune response.

7. The use according to Claim 6, wherein said autoimmune diseases are one of selected from the group consisting of rheumatoid arthritis, collagen induced arthritis, multiple sclerosis, experimental autoimmune encephalomyelitis, insulin-dependent diabetes mellitus, experimental diabetes mellitus and uveitis.

8. The use according to Claim 7, wherein said autoimmune disease is rheumatoid arthritis.

9. The use according to Claim 6, wherein said antigen is type II collagen, S antigen, major basic protein, glutamic acid decarboxylase, or immunodominant peptide fragments thereof.

10. The use according to any one of Claim 6 to Claim 9, wherein said medicament is suitable for administration to human, rats, mice and monkeys.

11. The use according to any one of Claim 6 to Claim 9, wherein said particles have a size of less than about 500nm.

12. Use of a composition for preparing a medicament suitable for oral administration for inducing tolerance for autoimmune diseases, which comprises as an active ingredient particles of biodegradable polymers entrapping an autoimmune antigen in an amount effective to induce tolerance against autoimmune response, and said particles of biodegradable polymers capable of reducing autoimmune response, wherein said particles have a diameter ranging from one hundred nanometer to several thousand nanometers, wherein said biodegradable polymers are poly (DL-lactide-co-glycolide), polylactides or polyglycolides, wherein a single oral dose of said particles is suitable to be administered to induce tolerance against autoimmune response.

## Patentansprüche

1. Verwendung von Teilchen von bioabbaubaren Polymeren, die in der Lage sind, eine Autoimmunantwort zu reduzieren, zur Herstellung eines zur oralen Verabreichung geeigneten Medikaments zur Behandlung von Autoimmunerkrankungen, wobei es sich bei den bioabbaubaren Polymeren um Poly(DL-lactid-co-glycolid), Polylactide oder Polyglycolide handelt, wobei eine einzelne orale Dosis der Teilchen dazu geeignet ist, zum Herbeiführen einer Toleranz gegen eine Autoimmunantwort verabreicht zu werden.

2. Verwendung nach Anspruch 1, wobei es sich bei den Autoimmunerkrankungen um eine von durch Th1 vermittelten oder durch T-Zellen vermittelten Autoimmunerkrankungen, ausgewählt aus der Gruppe, bestehend aus rheumatoider Arthritis, insulinabhängiger Diabetes mellitus, Uveitis, multipler Sklerose, Autoimmunthyroiditis, Autoimmunhepatitis, interstitieller Pneumonitis und Glomerulonephritis, und deren entsprechenden Erkrankungen in Tiermodellen handelt.

3. Verwendung nach Anspruch 2, wobei es sich bei der Autoimmunerkrankung um rheumatoide Arthritis handelt.

4. Verwendung nach einem der Ansprüche 1 bis 3, wobei das Medikament zur Verabreichung an Menschen, Ratten, Mäuse und Affen geeignet ist.

5. Verwendung nach einem der Ansprüche 1 bis 3, wobei die Teilchen eine Größe von weniger als etwa 500 nm aufweisen.

6. Verwendung von Teilchen von bioabbaubaren Polymeren, die ein Autoimmunantigen aufnehmen, zur Herstellung eines zur oralen Verabreichung geeigneten Medikaments zur Behandlung von Autoimmunerkrankungen, wobei die Teilchen von bioabbaubaren Polymeren in der Lage sind, eine Immunantwort zu reduzieren, wobei es sich bei den bioabbaubaren Polymeren um Poly(DL-lactid-co-glycolid), Polylactide oder Polyglycolide handelt, wobei eine einzelne orale Dosis der Teilchen dazu geeignet ist, zum Herbeiführen einer Toleranz gegen eine Autoimmunantwort verabreicht zu werden.

7. Verwendung nach Anspruch 6, wobei es sich bei den Autoimmunerkrankungen um eine von solchen, ausgewählt aus der Gruppe, bestehend aus rheumatoider Arthritis, durch Collagen herbeigeführter Arthritis, multipler Sklerose, experimenteller Enzephalomyelitis, insulinabhängiger Diabetes mellitus, experimenteller Diabetes mellitus und Uveitis, handelt.

8. Verwendung nach Anspruch 6, wobei es sich bei der Autoimmunerkrankung um rheumatoide Arthritis handelt.

9. Verwendung nach Anspruch 6, wobei es sich bei dem Antigen um Collagen Typ II, S-Antigen, Hauptbasisprotein, Glutaminsäuredecarboxylase oder immundominante Peptidfragmente davon handelt.

10. Verwendung nach einem der Ansprüche 6 bis 9, wobei das Medikament zur Verabreichung an Menschen, Ratten, Mäuse und Affen geeignet ist.

11. Verwendung nach einem der Ansprüche 6 bis 9, wobei die Teilchen eine Größe von weniger als etwa 500 nm aufweisen.

12. Verwendung einer Zusammensetzung zur Herstellung eines zur oralen Verabreichung geeigneten Medikaments zum Herbeiführen einer Toleranz für Autoimmunerkrankungen, die als Wirkstoff Teilchen von bioabbaubaren Polymeren, die ein Autoimmunantigen aufnehmen, in einer zum Herbeiführen einer Toleranz gegen eine Autoimmunantwort wirksamen Menge umfasst, und wobei die Teilchen von bioabbaubaren Polymerem in der Lage sind, eine Immunantwort zu reduzieren, wobei die Teilchen einen Durchmesser im bereich von einhundert Nanometern bis mehreren Tausend Nanometern aufweisen, wobei es sich bei den bioabbaubaren Polymeren um Poly(DL-lactid-co-glycolid), Polylactide oder Polyglycolide handelt, wobei eine einzelne orale Dosis der Teilchen dazu geeignet ist, zum Herbeiführen einer Toleranz gegen eine Autoimmunantwort verabreicht zu werden.

## Revendications

1. Utilisation de particules de polymères biodégradables capables de réduire une réponse auto-immune pour préparer un médicament adapté à une administration orale pour traiter des maladies auto-immunes, dans laquelle lesdits polymères biodégradables sont des poly(DL-lactide-co-glycolide), polylactides ou polyglycolides, dans laquelle une dose orale unique desdites particules est adaptée pour être administrée afin d'induire une tolérance vis-à-vis d'une réponse auto-immune.

2. Utilisation selon la revendication 1, dans laquelle lesdites maladies auto-immunes sont l'une parmi des maladies auto-immunes régulées par Th1 ou régulées par cellules T choisies parmi le groupe constitué de la polyarthrite rhumatoïde, diabète insulino-dépendant, uvéite, sclérose en plaques, thyroïdite auto-immune, hépatite auto-immune, pneumonie interstitielle et glomérulonéphrite, et leurs maladies correspondantes chez des modèles animaux.

3. Utilisation selon la revendication 2, dans lequel ladite maladie auto-immune est la polyarthrite rhumatoïde.

4. Utilisation selon l'une quelconque des revendications 1 à 3, dans laquelle ledit médicament est adapté à une administration à l'homme, des rats, des souris et des singes.

**5.** Utilisation selon l'une quelconque des revendications 1 à 3, dans laquelle lesdites particules ont une taille inférieure à environ 500 nm.

**6.** Utilisation de particules de polymères biodégradables renfermant un antigène auto-immune pour préparer un médicament adapté à une administration orale pour traiter des maladies auto-immunes, dans laquelle lesdites particules de polymères biodégradables sont capables de réduire une réponse auto-immune, dans laquelle lesdits polymères biodégradables sont des poly(DL-lactide-co-glycolide), polylactides ou polyglycolides, dans laquelle une dose orale unique desdites particules est adaptée pour être administrée afin d'induire une tolérance vis-à-vis d'une réponse auto-immune.

**7.** Utilisation selon la revendication 6, dans laquelle lesdites maladies auto-immunes sont l'une choisie parmi le groupe constitué de la polyarthrite rhumatoïde, arthrite induite par collagène, sclérose en plaques, encéphalomyélite auto-immune, diabète insulino-dépendant, diabète expérimental et uvéite.

**8.** Utilisation selon la revendication 7, dans laquelle ladite maladie auto-immune est la polyarthrite rhumatoïde.

**9.** Utilisation selon la revendication 6, dans laquelle ledit antigène est un collagène de type II, un antigène S, une protéine basique majeure, une acide glutamique décarboxylase, ou des fragments peptidiques immuno-dominant de ceux-ci.

**10.** Utilisation selon l'une quelconque des revendications 6 à 9, dans laquelle ledit médicament est adapté pour une administration à l'homme, des rats, des souris et des singes.

**11.** Utilisation selon l'une quelconque des revendications 6 à 9, dans laquelle lesdites particules ont une taille inférieure à environ 500 nm.

**12.** Utilisation d'une composition pour préparer un médicament adapté à une administration orale pour induire une tolérance à des maladies auto-immunes, qui comporte, en tant qu'ingrédient actif, des particules de polymères biodégradables renfermant un antigène auto-immune selon une quantité efficace pour induire une tolérance vis-à-vis d'une réponse auto-immune, et lesdites particules de polymères biodégradables capables de réduire une réponse auto-immune, dans laquelle lesdites particules ont un diamètre dans la plage d'une centaine de nanomètres à plusieurs milliers de nanomètres, dans laquelle lesdits polymères biodégradables sont des poly(DL-lactide-co-glycolide), polylactides ou polyglycolides, dans laquelle une dose orale unique desdites particules est adaptée pour être administrée afin d'induire une tolérance vis-à-vis d'une réponse auto-immune.

FIG. 1

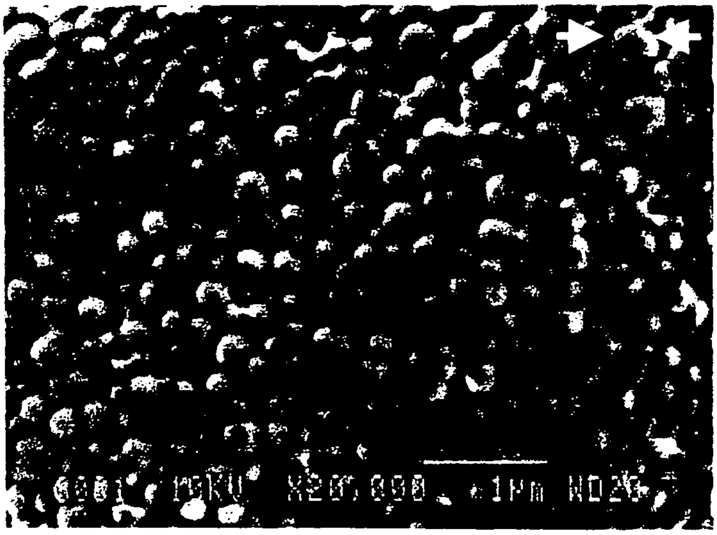

FIG. 2

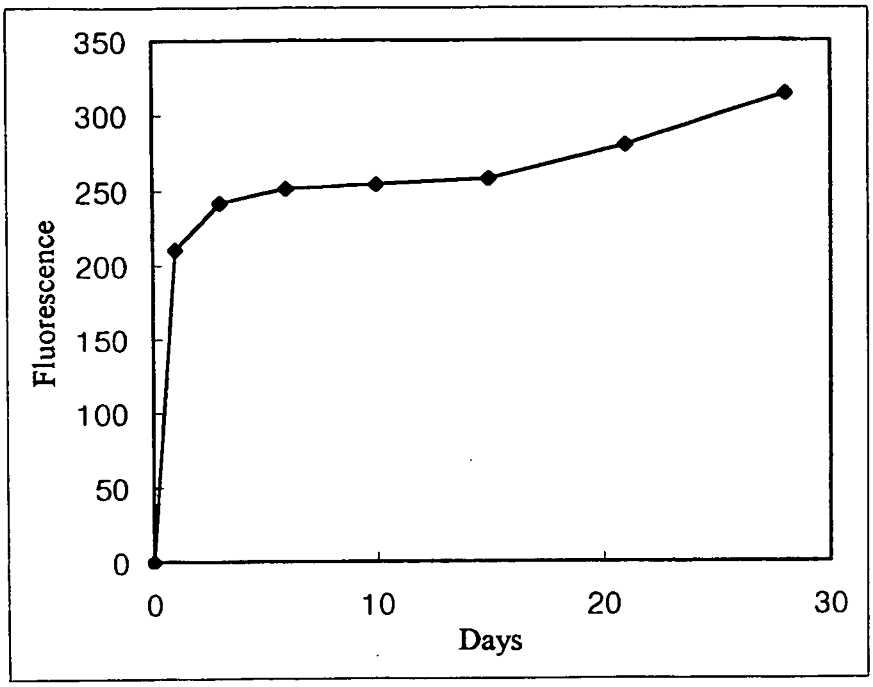

FIG. 3

(a)

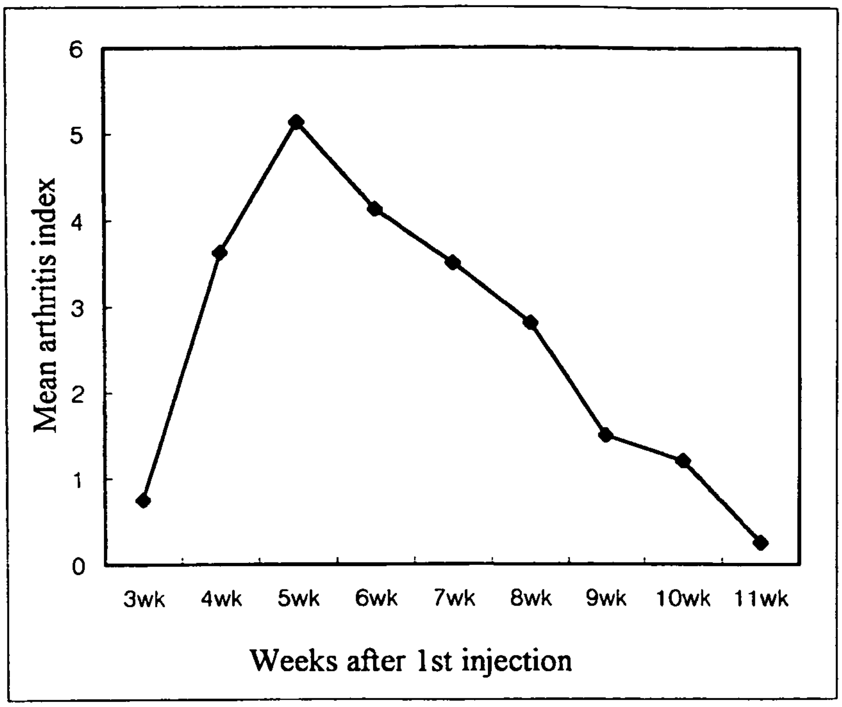
6
5
4
3
2
1
0
Mean arthritis index
3wk
4wk
5wk
6wk
7wk
8wk
9wk
10wk
11wk
Weeks after 1st injection

(b)

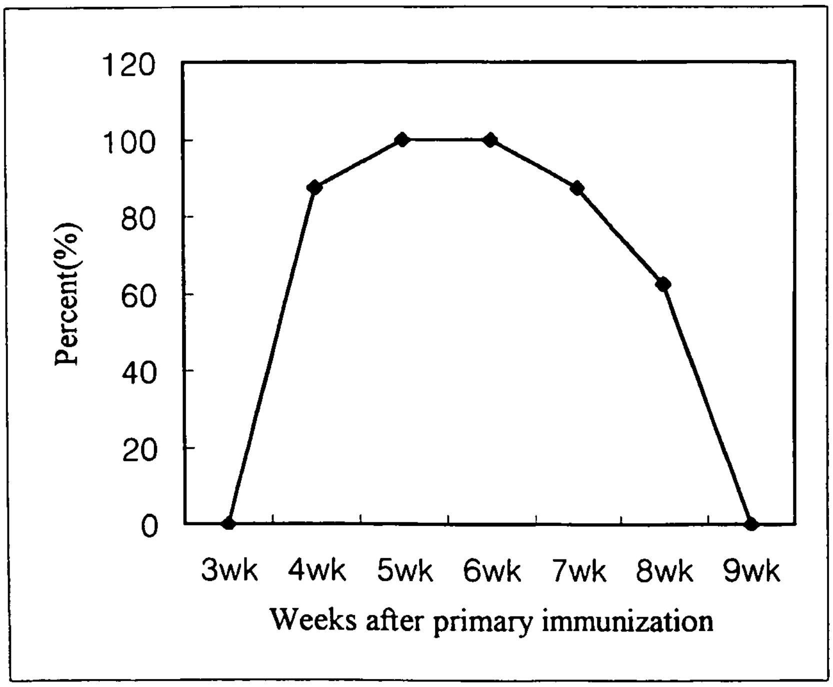
120
100
80
60
40
20
0
Percent(%)
3wk
4wk
5wk
6wk
7wk
8wk
9wk
Weeks after primary immunization

FIG. 4

(a)

(b)

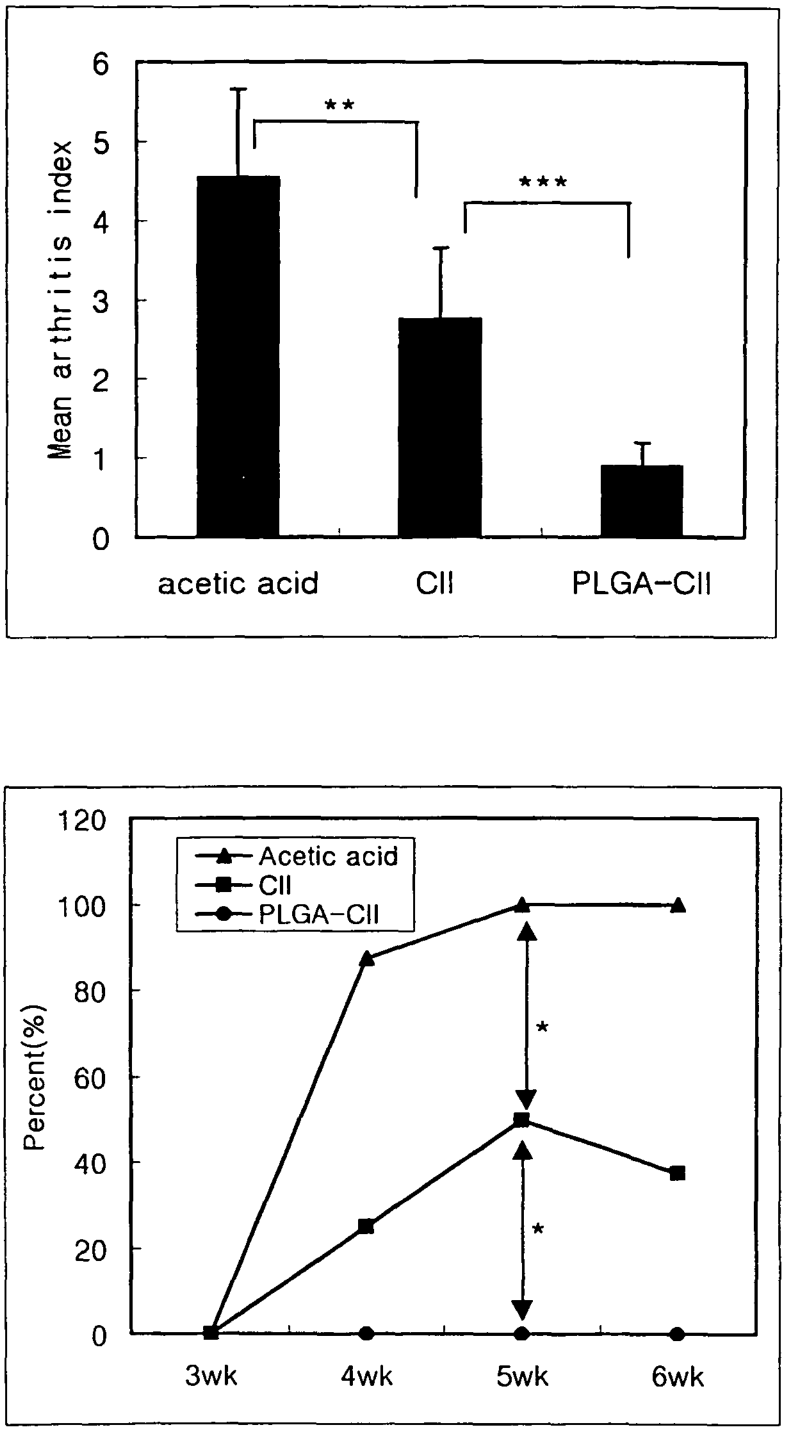
Mean arthritis index
6
5
4
3
2
1
0
**
***
acetic acid
CII
PLGA-CII
120
100
80
60
40
20
0
Percent(%)
Acetic acid
CII
PLGA-CII
*
*
3wk
4wk
5wk
6wk

FIG. 4

(c)

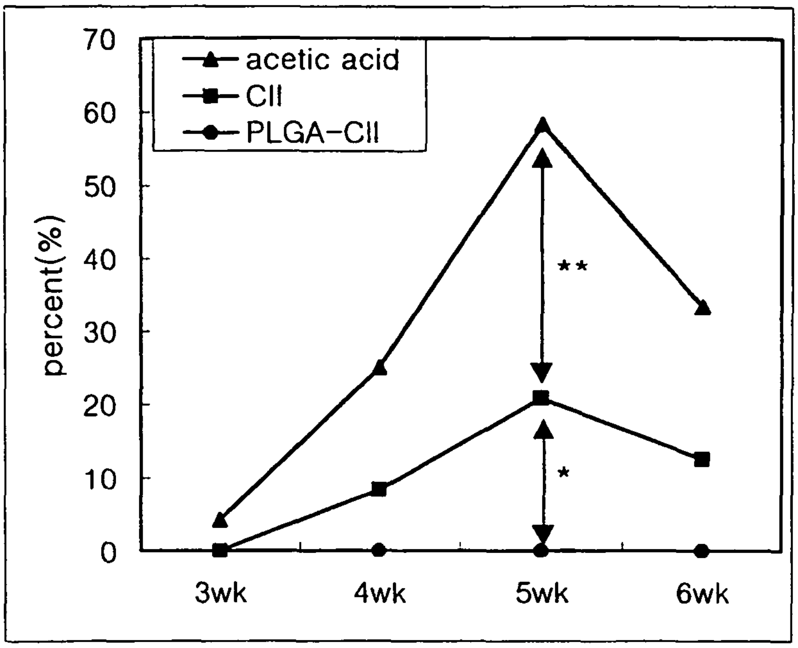
70
60
50
40
30
20
10
0
percent(%)
acetic acid
CII
PLGA-CII
**
*
3wk
4wk
5wk
6wk

FIG. 5

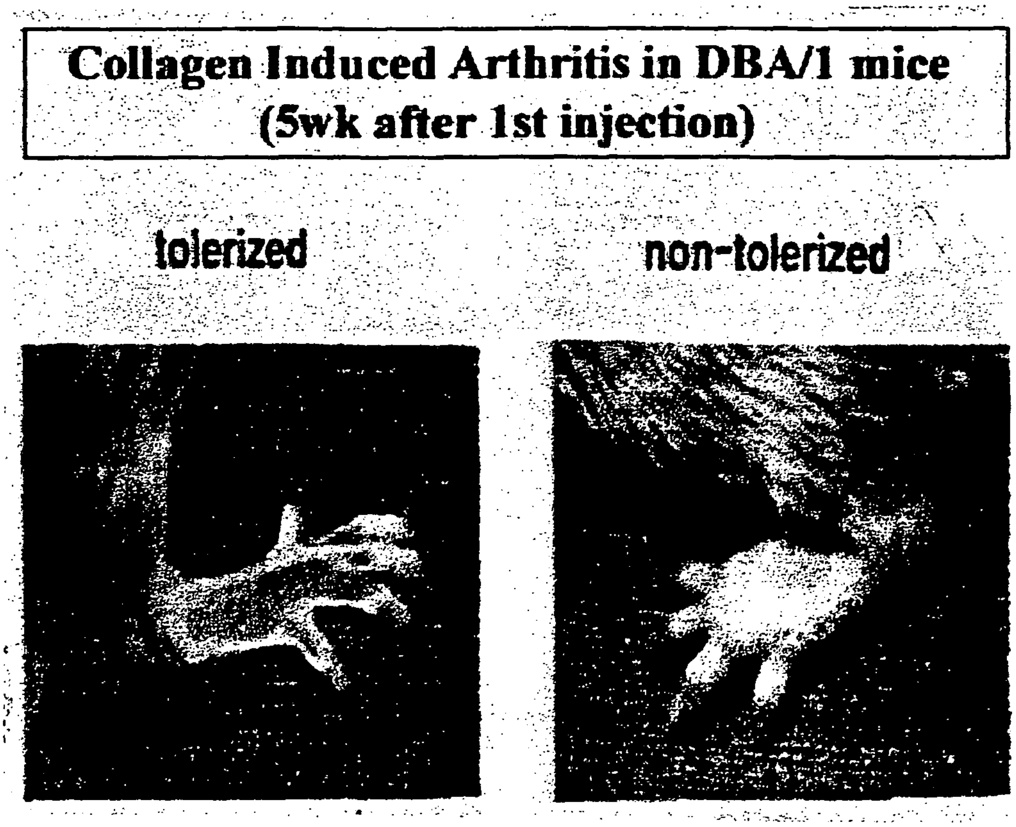
Collagen Induced Arthritis in DBA/1 mice
(5wk after 1st injection)
tolerized
non-tolerized

FIG. 6

(a)

(b)

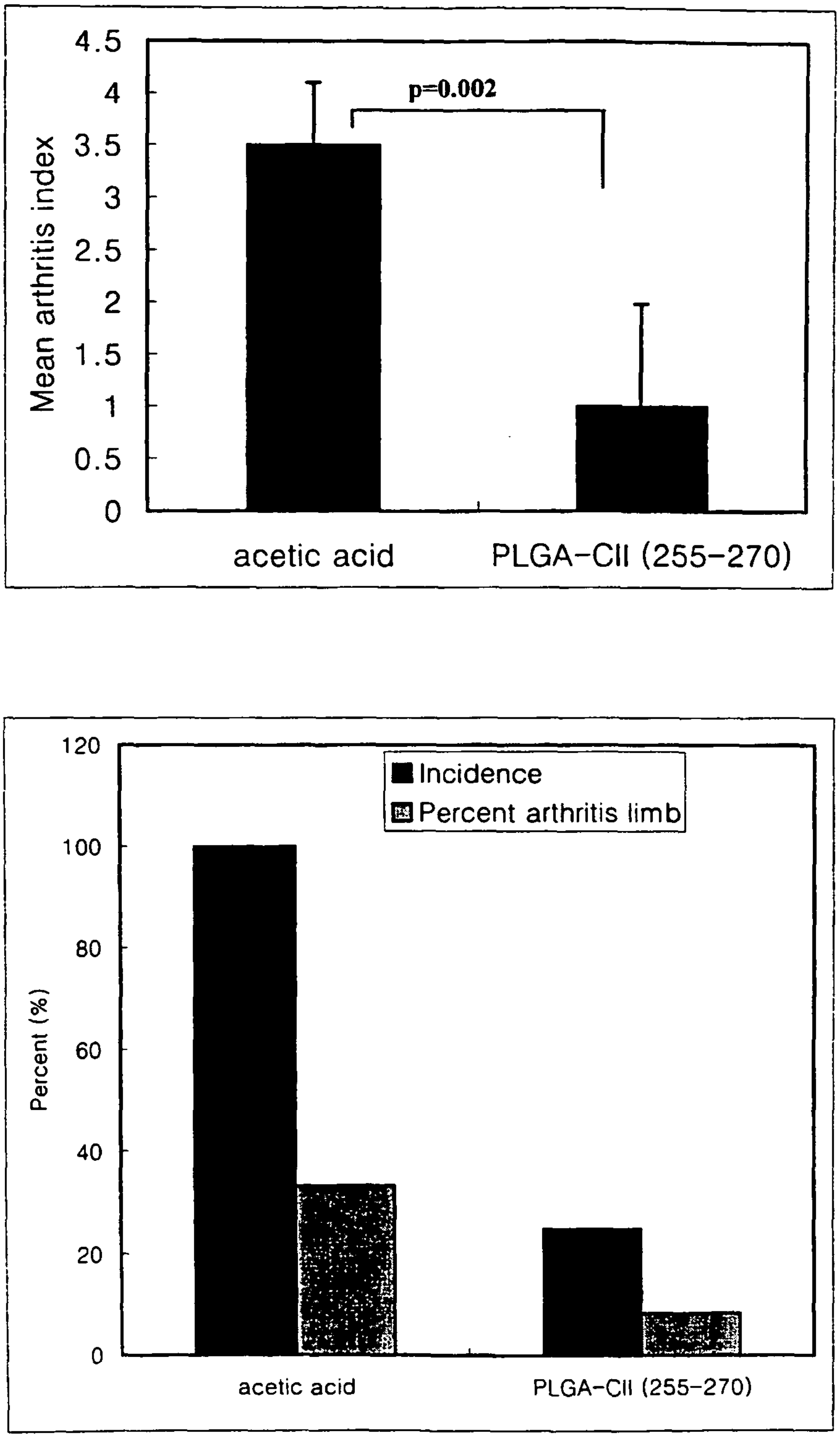
4.5
4
3.5
3
2.5
2
1.5
1
0.5
0
Mean arthritis index
p=0.002
acetic acid
PLGA-CII (255-270)
120
100
80
60
40
20
0
Percent (%)
Incidence
Percent arthritis limb
acetic acid
PLGA-CII (255-270)

# FIG. 7

(a)

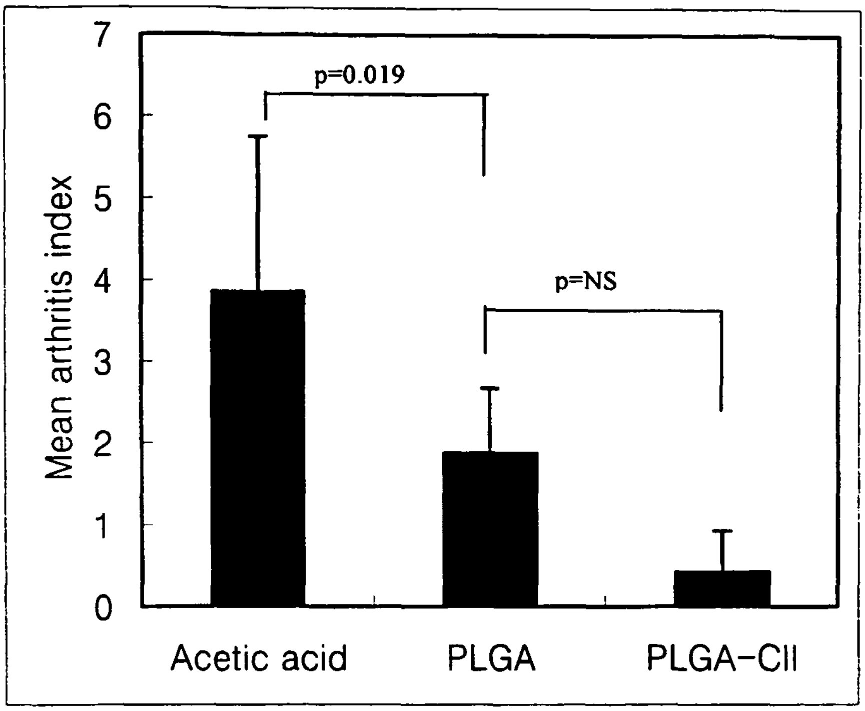
Mean arthritis index
0
1
2
3
4
5
6
7
p=0.019
p=NS
Acetic acid
PLGA
PLGA-CII

(b)

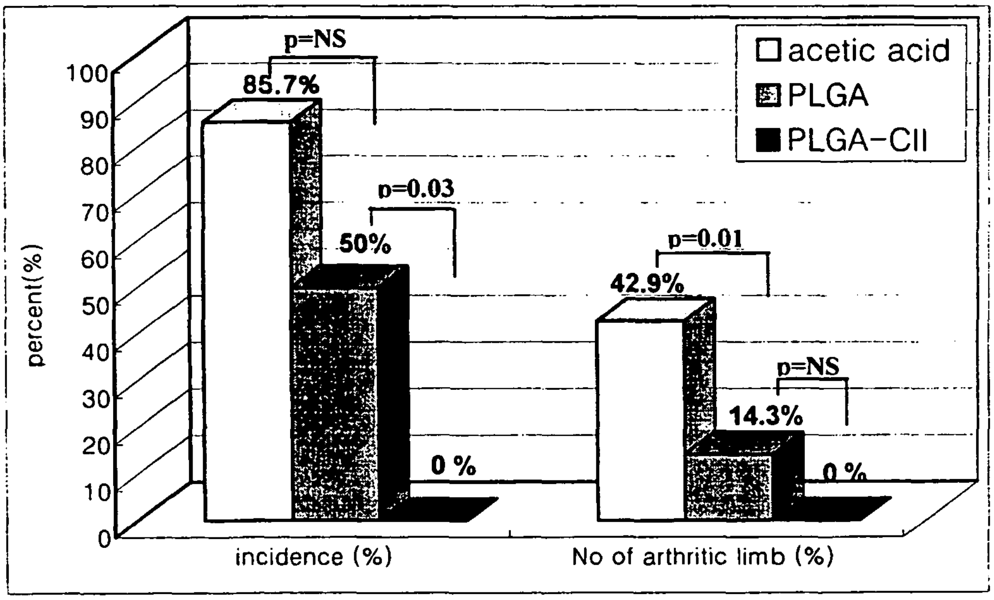
percent(%)
0
10
20
30
40
50
60
70
80
90
100
p=NS
85.7%
p=0.03
50%
0 %
p=0.01
42.9%
p=NS
14.3%
0 %
acetic acid
PLGA
PLGA-CII
incidence (%)
No of arthritic limb (%)

# FIG. 8

(a)

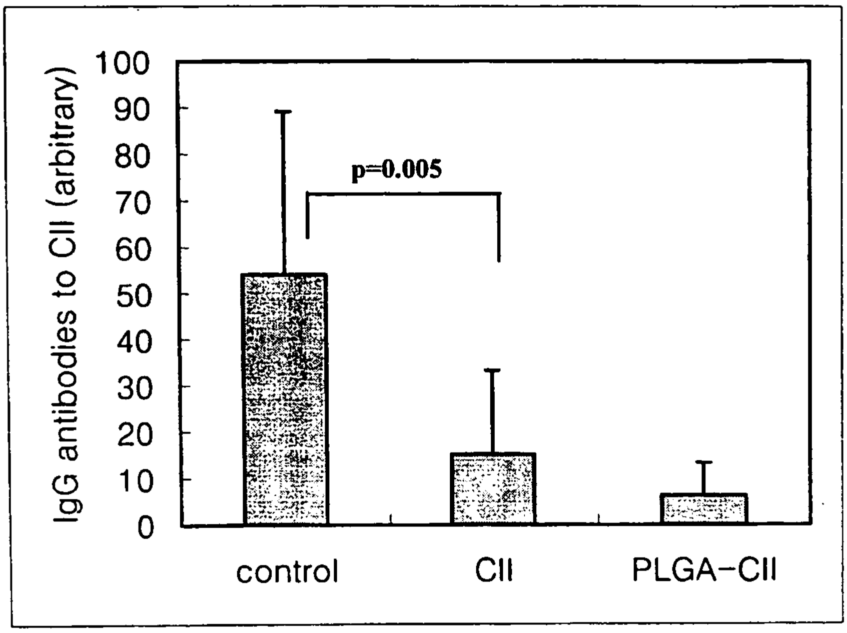
IgG antibodies to CII (arbitrary)
100
90
80
70
60
50
40
30
20
10
0
p=0.005
control
CII
PLGA-CII

(b)

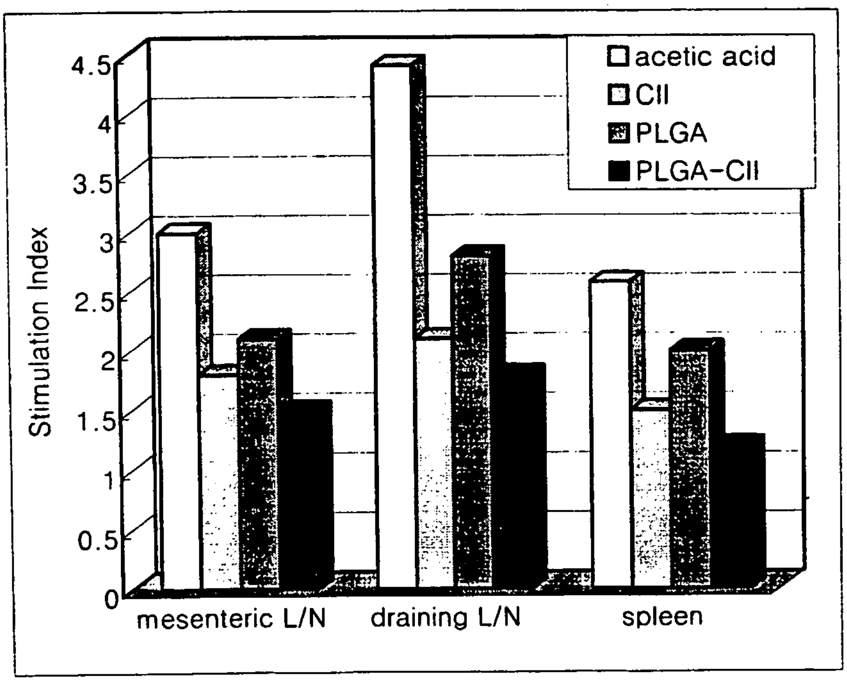
Stimulation Index
4.5
4
3.5
3
2.5
2
1.5
1
0.5
0
acetic acid
CII
PLGA
PLGA-CII
mesenteric L/N
draining L/N
spleen

FIG. 9

(a)

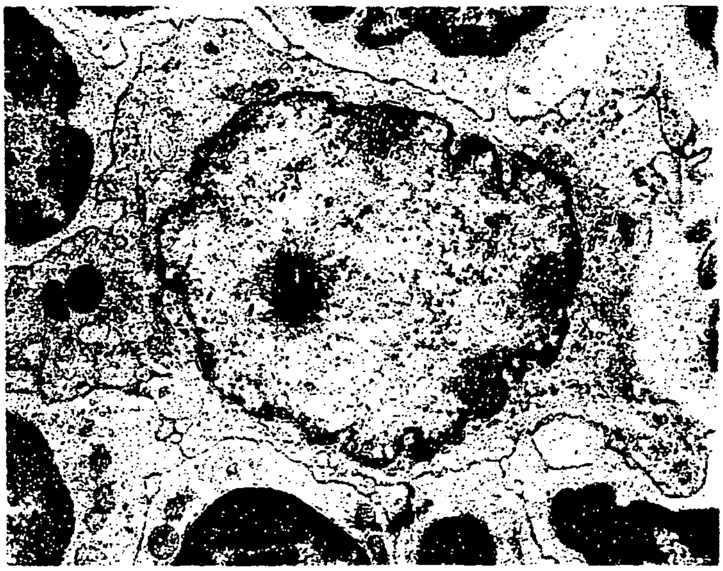

FIG. 9

(b)

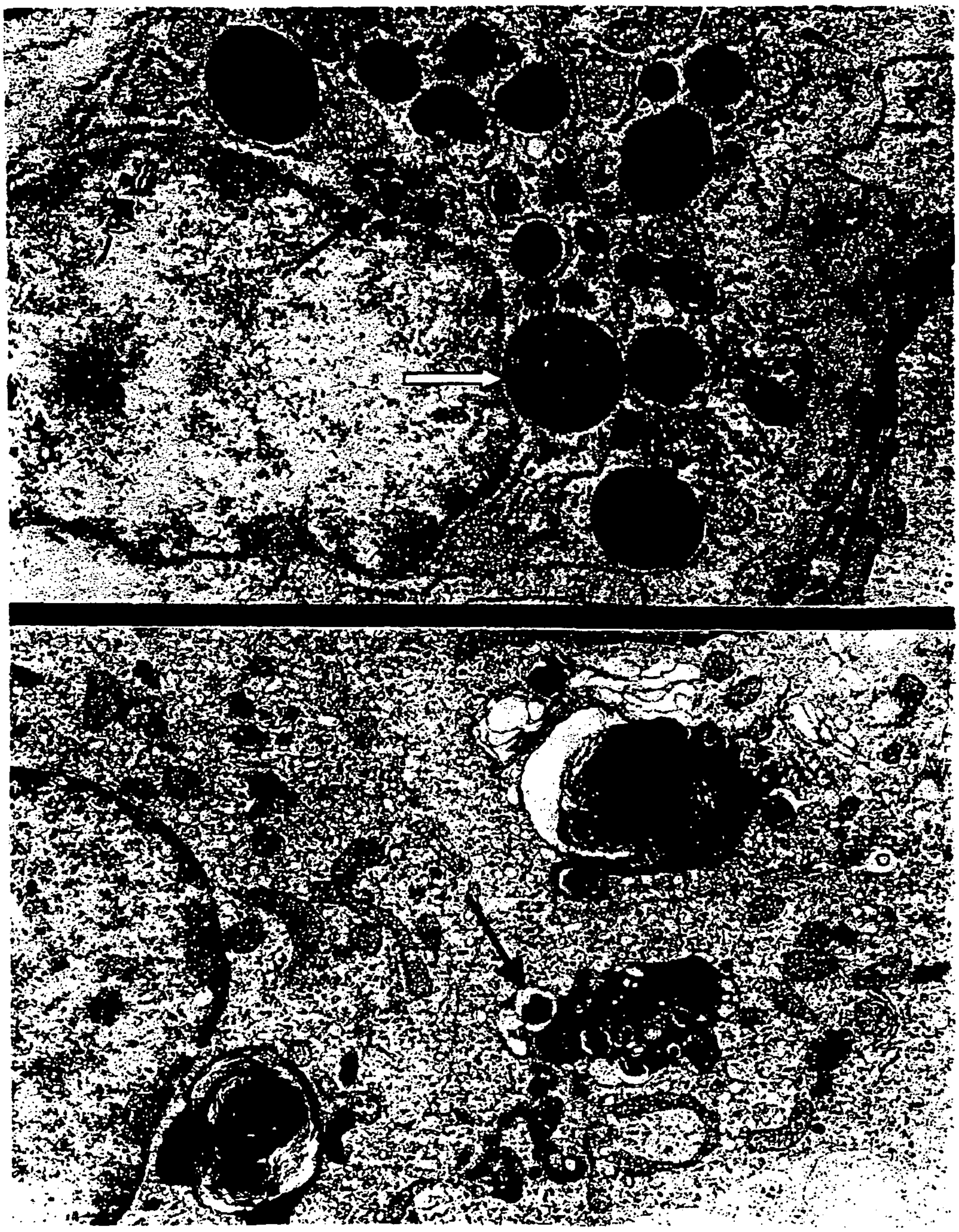

FIG. 10

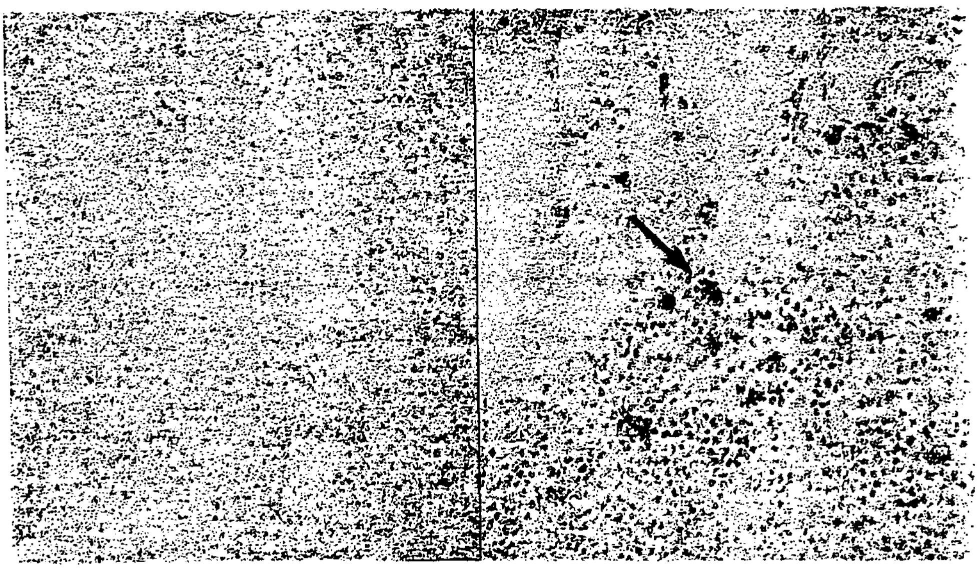

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9909956 A
- KR 9900000 W


### Non-patent literature cited in the description


- **T. URATA et al.** *Journal of Controlled Release,* 1999, vol. 58, 133-141
- **T. UCHIDA.** *Chem. Phar. Bull.,* 1997, vol. 45 (9), 1539-1543
- **WEINER HL ; MACKIN GA ; MATSUI M et al.** Double-blind pilot trial of oral tolerization with myelin antigens in multiple sclerosis. *Science,* 1993, vol. 259, 1321-1324
- **TRENTHAM DE ; DYNESIUS-TRENTHAM RA ; ORAV EJ et al.** Effects of oral administration of type II collagen on rheumatoid arthritis. *Science,* 1993, vol. 261, 1727-1730
- **ZHANG ZJ ; DAVIDSON L ; EISENBARTH G et al.** Suppression of diabetes in non-obese diabetic mice by oral administration of porcine insulin. *Proc Natl Acad Sci USA,* 1991, vol. 88, 10252-10256
- **NUSSENBLATT RB ; CASPI RR ; MAHDI R et al.** Inhibition of S-antigen induced experimental autoimmune uveoretinitis by oral induction of tolerance with S-antigen. *J Immunol,* 1990, vol. 144, 1689-1695
- **SUN JB ; HOLMGREN J ; CZERKINSKY C.** Cholera toxin B subunit: an efficient transmucosal carrier-delivery system for induction of peripheral immunological tolerance. *Proc Natl Acad Sci USA,* 1994, vol. 91, 10795-10799
- **MELAMED D ; FRIEDMAN A.** In vivo tolerization of Th1 lymphocytes following a single feeding with ovalbumin: anergy in the absence of suppression. *Eur J Immunol,* 1994, vol. 24, 1974-1981
- **MELAMED D ; FISHMAN-LOVELL J ; UNI Z et al.** Peripheral tolerance of Th2 lymphocytes induced by continuous feeding of ovalbumin. *Int Immunol,* 1996, vol. 8, 717-724
- **STOKES CR ; SWARBRICK ET ; SOOTHILL JF.** Genetic differences in immune exclusion and partial tolerance to ingested antigens. *Clin Exp Immunol,* 1983, vol. 52, 678-684
- **MOREAU MC ; COSTE M ; GABORIAU V et al.** Oral tolerance to ovalbumin in mice: effect of some parameters on the induction and persistence of the suppression of systemic IgE and IgG antibody responses. *Adv Exp Med Biol,* 1995, vol. 371, 1229-1234
- **PENG HJ ; CHANG ZN ; HAN SH et al.** Chemical denaturation of ovalbumin abrogates the induction of oral tolerance of specific IgG antibody and DTH responses in mice. *Scand J Immunol,* 1995, vol. 42, 297-304
- **PENG HJ ; TURNER MW ; STROBEL S.** The generation of a 'tolerogen' after the ingestion of ovalbumin is time-dependent and unrelated to serum levels of immunoreactive antigen. *Clin Exp Immunol,* 1990, vol. 81, 510-515
- **HANSON LA ; TELEMO E ; WIEDERMANN U et al.** Sensitization and development of tolerance via the gut. *Pediatr Allergy Immunol,* 1993, vol. 4, 16-20
- **COUVREUR P ; PUISIEUX F.** Nano- and microparticles for the delivery of polypeptides and proteins. *Adv. Drug Del. Rev,* 1993, vol. 10, 141
- **LAVELLE EC ; SHARIF S ; THOMAS NW et al.** The importance of gastrointestinal uptake of particles in the design of oral delivery systems. *Adv. Drug Del. Rev,* 1995, vol. 18, 5
- **ANDRIANOV AK ; PAYNE LG.** Polymeric carriers for oral uptake of microparticulates. *Adv. Drug Del. Rev,* 1998, vol. 34, 155
- **FLORENCE AT.** The oral absorption of micro- and nanoparticulates: neither exceptional nor unusual. *Pharm. Res,* 1997, vol. 14, 259
- **JANI P ; HALBERT GW ; LANGRIDGE J et al.** Particle Uptake by the Rat Gastrointestinal Mucosa: Quntitation and Particle Size Dependency. *J. Pharm. Pharmacol,* 1990, vol. 42, 821
- **JANI P ; FLORENCE AT ; MCCARTHY DE.** Further Histological evidence of the gastrointestinal absorption of polystyrene nanospheres in the rat. *Int. J. Pharm,* 1992, vol. 84, 245
- **JEPSON MA ; CLARK MA ; FOSTER N et al.** Targeting to intestinal M cells. *J. Anat.,* 1996, vol. 189, 507-516
- **MOLDOVEANU Z ; NOVAK M ; HUANG W-Q et al.** Oral immunization with influenza in biodegradable microspheres. *J. Infect. Dis,* 1993, vol. 167, 84-90

- **SMITH MW ; THOMAS NW ; KENKINS PG et al.** Selective transport of microparticles across Peyer's patch follicle-associated M cells from mice and rats. *Exp Physiol.,* 1995, vol. 80, 735-743
- **KREY A ; GIANNASCA KT ; WELTZEIN R et al.** Role of the glycocolyx in regulating access of microparticles to apical plasma membranes of intestinal epithelial cells: Implications for microbial attachment and oral vaccine targeting. *J. Exp. Med,* 1996, vol. 184, 1045-1059
- **TEITELBAUM D ; ARNON R ; SELA M.** Immunomodulation of experimental autoimmune encephalitis by oral administration of copolymer 1. *Proc Natl Acad Sci USA,* 1999, vol. 96, 3842-3847
- **FRIDKIS-HARELI M ; ROSNOLEIC E ; FUGGER L et al.** *Synthetic amino acid copolymers that bind to HLA-DR proteins and inhibit type II collagen-reactive T cell clones*
- **ITO HO ; SO T ; UEDA T et al.** Prevention of collagen induced arthritis (CIA) by treatment with polyethylene glycol-conjugated type II collagen; distinct tolerogenic property of the conjugated collagen from the native one. *Clin Exp Immunol,* 1997, vol. 108, 213-219
- **HERITAGE PL ; LOOMES LM ; JIANXIONG J et al.** Novel polymer-grafted starch microparticles for mucosal delivery of vaccines. *Immunology,* 1996, vol. 88, 162-168
- **TRENTHAM DE ; TOWNES AS ; KANG AH.** Autoimmunity to type II collagen: An experimental model of arthritis. *J Exp Med,* 1977, vol. 146, 857-868
- **COURTENAY JS ; DALLMAN MJ et al.** Immunisation against heterologous type II collagen induces arthritis in mice. *Nature,* 1980, vol. 283, 666-668
- **TRENTHAM DE ; DYNESIUS RA et al.** Cellular sensitivity to collagen in rheumatoid arthritis. *N Eng J Med,* 1978, vol. 299, 327-332
- **RUDOLPHI U ; RZEPKA R ; BATSFORD S et al.** The B cell repertoire of patients with rheumatoid arthritis. Increased frequencies of IgG+ and IgA+ B cells specific for mycobacterial heat-shock protein 60 or human type II collagen in synovial fluid and tissue. *Arthritis Rheum,* 1997, vol. 40, 1409-1419
- **TARKOWSKI A ; KLARESKOG L ; CARLSTEN H et al.** Secretion of antibodies to types I and II collagen by synovial tissue cells in patients with rheumatoid arthritis. *Arthritis Rheum,* 1989, vol. 32, 1087-1092
- **BARNETT ML ; KREMER JM ; CLAIR EW et al.** Treatment of rheumatoid arthritis with oral type II collagen : Results of a multicenter, double-blind, placebo-controlled trial. *Arthritis Rheum,* 1998, vol. 41, 290-297
- **SIEPER J ; KARY S ; SORENSEN H et al.** Oral type II collagen treatment in early rheumatoid arthritis : a double-blind, placebo-controlled, randomized trial. *Arthritis Rheum,* 1996, vol. 39, 41-51
- **KHARE SD ; KRCO CJ ; GRIFFITHS MM et al.** Oral administration of an immunodominant human collagen peptide modulates collagen-induced arthritis. *J Immunol,* 1995, vol. 155, 3653-3659
- **KRCO CJ ; PAWELSKI J ; HARDERS J et al.** Characterization of the antigenic structure of human type II collagen. *J Immunol,* 1996, vol. 156, 2761-2768
- **YU M ; JOHNSON JM ; TUOHY VK.** A predictable sequential determinant spreading cascade invariably accompanies progression of experimental autoimmune encephalomyelitis: a basis for peptide specific therapy after onset of clinical disease. *J Exp Med,* 1996, vol. 183, 1777-1788
- **TUOHY VK ; YU M, L et al.** Spontaneous regression of primary autoreactivity during chronic progression of experimental autoimmune encephalomyelitis and multiple sclerosis. *J Exp Med,* 1999, vol. 189, 1033-42
- **VRABEC TR ; GREGERSON DS ; DUA HS et al.** Inhibition of experimental autoimmune uveoretinitis by oral administration of S-antigen and synthetic peptides. *Autoimmunity,* 1992, vol. 12, 175-84
- **STUART JM ; CREMER MA ; KANG AH et al.** Collagen induced arthritis in rats: evaluation of early immunologic events. *Arthrits Rhem,* 1978, vol. 22, 1344
- **DESAI MP ; LABHASETWAR V ; AMIDON GL et al.** Gastrointestinal Uptake of Biodegradable Microparticles: Effect of Particle Size. *Pharm. Res,* 1996, vol. 13, 1838
- **KAKIMOTO K ; KATSUKI M ; HIROFUJII T et al.** Isolation of T cell line capable of protecting mice against collagen induced arthritis in mice. *J Immunol,* 1988, vol. 140, 78-83
- **CHEN SL ; WHITELEY PJ ; FREED DC et al.** Responses of NOD congenic mice to a glutamic acid decarboxylase-derived peptide. *J Autoimmunity,* 1994, vol. 7, 635-41